(19) Europäisches Patentamt
European Patent Office
Office européen des brevets

(11) EP 0 707 000 A1

(12) EUROPEAN PATENT APPLICATION
published in accordance with Art. 158(3) EPC

(43) Date of publication:
**17.04.1996 Bulletin 1996/16**

(21) Application number: **94918586.2**

(22) Date of filing: **28.06.1994**

(51) Int. Cl.$^6$: **C07D 213/81**, C07D 213/83, C07D 233/90, C07D 277/56, C07D 417/12, C07D 401/12, C07D 403/12, C07D 285/06, C07D 231/16, A01N 43/40, A01N 43/50

(86) International application number: **PCT/JP94/01041**

(87) International publication number:
**WO 95/01340 (12.01.1995 Gazette 1995/03)**

(84) Designated Contracting States:
**CH DE FR GB IT LI NL**

(30) Priority: **29.06.1993 JP 159196/93**
**05.10.1993 JP 249597/93**
**18.03.1994 JP 49242/94**
**16.06.1994 JP 134360/94**

(71) Applicant: **NISSAN CHEMICAL INDUSTRIES, LIMITED**
**Chiyoda-ku Tokyo 101 (JP)**

(72) Inventors:
- **AKIYAMA, Shigeaki,**
  **Nissan Chemical Ind., Ltd.,**
  **Funabashi-shi, Chiba-ken 274 (JP)**
- **TAKEYAMA, Toshiaki,**
  **Nissan Chemical Ind., Ltd.,**
  **Funabashi-shi, Chiba-ken 274 (JP)**
- **SUZUKI, Hiroyuki,**
  **Nissan Chemical Industries Ltd.**
  **Funabashi-shi, Chiba-ken 274 (JP)**
- **YASUMI, Yoshiaki,**
  **Nissan Chemical Industries Ltd.**
  **Funabashi-shi, Chiba-ken 274 (JP)**
- **WATANABE, Junichi,**
  **Nissan Chemical Ind., Ltd.,**
  **Funabashi-shi, Chiba-ken 274 (JP)**
- **NAKAJIMA, Yasuyuki,**
  **Nissan Chemical Ind., Ltd.,**
  **Funabashi-shi, Chiba-ken 274 (JP)**
- **OHYA, Hiroshi,**
  **Nissan Chem. Ind. Ltd.**
  **Shiraoka**
  **ShiShiraoka-cho, Saitama-ken 349-02 (JP)**
- **SASABE, Shigeru,**
  **Nissan Chem.Ind. Ltd.**
  **Shiraoka**
  **Shiraoka-cho Saitama-ken 349-02 (JP)**
- **NISHIOKA, Masanori,**
  **Nissan Chem. Ind.Ltd.**
  **Shiraoka**
  **Shiraoka-cho Saitama-ken 349-02 (JP)**
- **FURUSATO, Takashi,**
  **Nissan Chem. Ind.Ltd**
  **Shiraoka**
  **Shiraoka-cho Saitama-ken 349-02 (JP)**

(74) Representative: **Kolb, Helga, Dr. Dipl.-Chem. et al**
**Hoffmann, Eitle & Partner,**
**Patent-und Rechtsanwälte,**
**Arabellastrasse 4**
**D-81925 München (DE)**

# (54) ALDOXIME DERIVATIVE AND AGROHORTICULTURAL BACTERICIDE

(57) An aldoxime derivative represented by general formula (I), wherein A represents pyrazole, thiazole, etc.; B represents pyridine, pyrazole, thiazole, etc.; and X represents halogen or $S(O)_nR^{24}$. This compound is novel, has an excellent agrohorticultural bactericidal effect, and does not injure useful crops, thus being useful as an agrohorticultural bactericide.

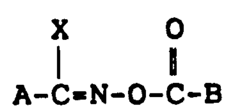

(I)

EP 0 707 000 A1

## Description

Technical Field

The present invention relates to novel aldoxime derivatives and agricultural and horticultural bactericides containing the same as an active ingredient.

Technical Background

Various fungicides have heretofore developed, but the potency of them could not be always satisfactory due to appearance of resistant strains and other reasons.

U. S. Patent No. 4,244,959 and Japanese Patent Application Laid-open No. Hei 4-134071 describe that some aldoxime derivatives exhibit bactericidal activity.

U. S. Patent No. 4,347,372 describes some aldoxime derivatives, but does not at all disclose the bactericidal activity.

The compounds described in the above-mentioned publications are not still satisfactory with respect to the potency, residual effect and the like. Thus, the development of agricultural and horticultural bactericides which are further more useful to plant diseases is desired.

Disclosure of the Invention

Under these circumstances, the present inventors have conducted investigations to develop compounds having excellent bactericidal activity, and have consequently found that aldoxime derivatives represented by formula [1] exhibit exellent bactericidal activity. This finding has led to the completion of the present invention.

That is, the present invention relates to aldoxime derivatives of formula [1]:

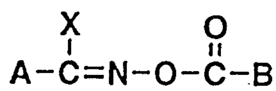

[1]

wherein X represents a halogen atom or $S(O)_nR^{24}$,

n is 0 or an integer of 1 to 2,

$R^{24}$ represents a $C_1$-$C_6$ alkyl group or an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

A represents

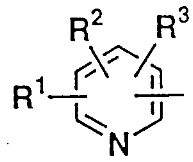 , 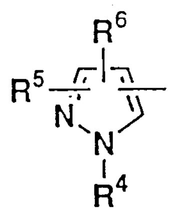 , 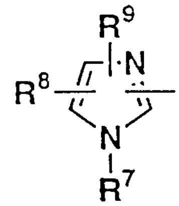 ,

A-1 A-2 A-3

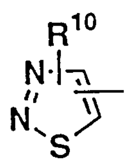 or 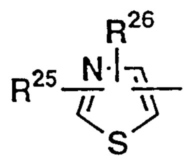

A-4 A-5

$R^4$ and $R^7$ independently represent a $C_1$-$C_6$ alkyl group, a $C_2$-$C_4$ alkenyl group, a $C_2$-$C_4$ alkynyl group, a $C_1$-$C_6$ haloalkyl group, an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), or an optionally substituted pyridyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 4, and the substituents may be the same or different),

$R^1$ to $R^3$, $R^5$, $R^6$, $R^8$ to $R^{10}$, $R^{25}$ and $R^{26}$ independently represent a hydrogen atom, a halogen atom, a $C_1$-$C_6$ alkyl group, a $C_2$-$C_4$ alkenyl group, a $C_2$-$C_4$ alkynyl group, a $C_1$-$C_6$ haloalkyl group, a $C_1$-$C_6$ alkoxy group, a $C_1$-$C_6$ alkoxycarbonyl group, a cyano group, an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), or an optionally substituted phenoxy group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

B represents a $C_1$-$C_6$ alkyl group, a $C_2$-$C_6$ alkenyl group, a $C_2$-$C_6$ alkynyl group, a $C_1$-$C_6$ haloalkyl group, a $C_1$-$C_6$ alkoxy group, a $C_1$-$C_6$ alkylamino group, an optionally substituted $C_3$-$C_6$ cycloalkyl group (the substituent thereof is a halogen atom, a $C_1$-$C_6$ alkyl group, a $C_1$-$C_6$ haloalkyl group or a $C_1$-$C_6$ alkoxy group, the number of the substituents is between 1 and 3, and the substituents may be the same or different), an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), an optionally substituted phenoxy group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), an optionally substituted phenylamino group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the

number of the substituents is between 1 and 5, and the substituents may be the same or different),

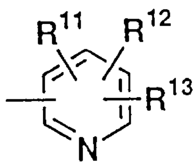 ,

B-1

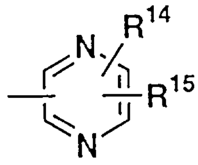 ,

B-2

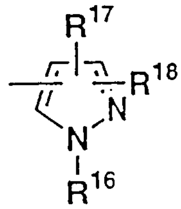 ,

B-3

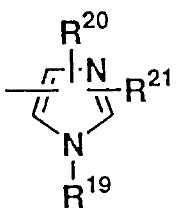 ,

B-4

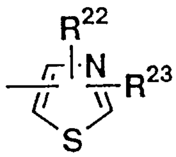 ,

B-5

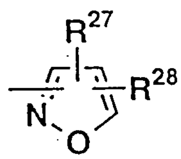 ,

B-6

or 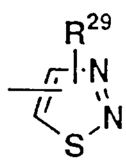 ,

B-7

$R^{16}$ and $R^{19}$ independently represent a $C_1$-$C_6$ alkyl group, a $C_2$-$C_4$ alkenyl group, a $C_2$-$C_4$ alkynyl group, a $C_1$-$C_6$ haloalkyl group or an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

$R^{11}$ to $R^{15}$, $R^{17}$, $R^{18}$, $R^{20}$ to $R^{23}$ and $R^{27}$ to $R^{29}$ independently represent a hydrogen atom, a halogen atom, a $C_1$-$C_6$ alkyl group, a $C_2$-$C_4$ alkenyl group, a $C_2$-$C_4$ alkynyl group, a $C_1$-$C_6$ haloalkyl group, a $C_1$-$C_6$ alkoxy group, an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different) or an optionally substituted phenoxy group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

provided that when the above-mentioned A is A-1, B is B-2, B-3, B-4, B-5, B-6 or B-7; and

agricultural and horticultural bactericides containing the aldoxime derivatives as an active ingredient.

Preferable examples of the aldoxime derivatives in the present invention are as follows:

aldoxime derivatives of formula [1] wherein A is A-2 or A-5;

aldoxime derivatives of formula [1] wherein A is A-2 or A-5 and B is B-1, B-3 or B-5;

aldoxime derivatives of formula [1] wherein A is A-2, B is B-1, B-3 or B-5, $R^4$ and $R^{16}$ are each a $C_1$-$C_6$ alkyl group, $R^5$, $R^6$, $R^{11}$ to $R^{13}$, $R^{17}$, $R^{18}$, $R^{22}$, $R^{23}$, $R^{25}$ and $R^{26}$ are independently a hydrogen atom, a halogen atom, a $C_1$-$C_6$ alkyl group, a $C_1$-$C_6$ haloalkyl group or a $C_1$-$C_6$ alkoxy group;

aldoxime derivatives of formula [1] wherein A is A-2 or A-5, B is B-1, B-3 or B-5, $R^4$ and $R^{16}$ are each a $C_1$-$C_6$ alkyl group, $R^5$, $R^6$, $R^{11}$ to $R^{13}$, $R^{17}$, $R^{18}$, $R^{22}$, $R^{23}$, $R^{25}$ and $R^{26}$ are independently a hydrogen atom, a halogen atom, a $C_1$-$C_6$ alkyl group, a $C_1$-$C_6$ haloalkyl group or a $C_1$-$C_6$ alkoxy group, and X is a halogen atom;

aldoxime derivatives of formula [1] wherein A is A-2, B is B-1, $R^4$ is a $C_1$-$C_6$ alkyl group, $R^5$, $R^6$ and $R^{11}$ to $R^{13}$ are independently a hydrogen atom, a halogen atom or a $C_1$-$C_6$ alkyl group, and X is a halogen atom;

aldoxime derivatives of formula [1] wherein A is A-2, B is B-3, $R^4$ and $R^{16}$ are each a $C_1$-$C_6$ alkyl group, $R^5$, $R^6$, $R^{17}$ and $R^{18}$ are independently a hydrogen atom, a halogen atom or a $C_1$-$C_6$ alkyl group, and X is a halogen atom;

aldoxime derivatives of formula [1] wherein A is A-2, B is B-5, $R^4$ is a $C_1$-$C_6$ alkyl group, $R^5$, $R^6$, $R^{22}$ and $R^{23}$ are independently a hydrogen atom, a halogen atom or a $C_1$-$C_6$ alkyl group, and X is a halogen atom;

aldoxime derivatives of formula [1] wherein A is A-5, B is B-1, $R^{11}$ to $R^{13}$, $R^{25}$ and $R^{26}$ are independently a hydrogen atom, a halogen atom, a $C_1$-$C_6$ alkyl group, and X is a halogen atom;

aldoxime derivatives of formula [1] wherein A is A-5, B is B-3, $R^{16}$ is a $C_1$-$C_6$ alkyl group, $R^{17}$, $R^{18}$, $R^{25}$ and $R^{26}$ are independently a hydrogen atom, a halogen atom or a $C_1$-$C_6$ alkyl group, and X is a halogen atom; and

aldoxime derivatives of formula [1] wherein A is A-5, B is B-5, $R^{22}$, $R^{23}$, $R^{25}$ and $R^{26}$ are independently a hydrogen atom, a halogen atom or a $C_1$-$C_6$ alkyl group, and X is a halogen atom.

Specific examples of the preferable compounds in the present invention are as follows.

(1) O-(5,6-dichloronicotinoyl)-5-chloro-4-(α-chloroformaldoxime)-1,3-dimethylpyrazole,
(2) O-(2,6-dichloropyridine-4-carbonyl)-5-chloro-4-(α-chloroformaldoxime)-1-ethyl-3-methylpyrazole,
(3) O-(2-chloro-6-methylpyridine-4-carbonyl)-5-chloro-4-(α-chloroformaldoxime)-1-ethyl-3-methylpyrazole,
(4) O-(5,6-dichloronicotinoyl)-4-chloro-5-(α-chloroformaldoxime)-1,3-dimethylpyrazole,
(5) O-(5-chloronicotinoyl)-4-chloro-5-(α-chloroformaldoxime)-1,3-dimethylpyrazole,
(6) O-(1,3-dimethylpyrazole-5-carbonyl)-5-chloro-4-(α-chloroformaldoxime)-1,3-dimethylpyrazole,
(7) O-(3-chloro-1-methylpyrazole-5-carbonyl)-5-chloro-4-(α-chloroformaldoxime)-1-ethyl-3-methylpyrazole,
(8) O-(2-chloro-4-methylthiazole-5-carbonyl)-5-chloro-4-(α-chloroformaldoxime)-1,3-dimethylpyrazole, and
(9) O-(2,6-dichloropyridine-4-carbonyl)-5-(α-chloroformaldoxime)-2,4-dimethylthiazole.

The present invention further relates to aldoxime derivatives of formula [2]:

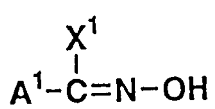

[2]

wherein $X^1$ represents a halogen atom, and $A^1$ represents A-2, A-3, A-4 or A-5, which are useful as intermediate compounds in the production of the above-mentioned aldoxime derivatives.

The substituents in formulas [1] and [2] of the compounds of the present invention are as follows.

The halogen atom includes fluorine, chlorine, bromine and iodine. Preferable are fluorine, chlorine and bromine. More preferable is a chlorine atom.

The alkyl group includes methyl, ethyl, n- or i-propyl, n-, s-, i- or t-butyl, pentyl and hexyl. Preferable are methyl, ethyl, n- or i-propyl, and n-, s-, i- or t-butyl.

The alkoxy group includes methoxy, ethoxy, n- or i-propoxy, n-, s-, i- or t-butoxy, pentoxy and hexoxy. Preferable are methoxy, ethoxy, n- or i-propoxy, and n-, s-, i- or t-butoxy.

The haloalkyl group includes fluoromethyl, difluoromethyl, trifluoromethyl, chloromethyl, bromomethyl, trifluoroethyl, trichloroethyl and trifluoropropyl.

Examples of the compounds of the formula [1] in the present invention are shown in Tables 1 to 3. However, the compounds of the present invention are not limited to these only.

Further, the compounds of the present invention include two types of isomers, E and Z (syn and anti), and the present invention encompasses these isomers too.

In the following tables, Ph indicates a phenyl group, Et does an ethyl group, Pr does a propyl group, Bu does a butyl group, Pen does a pentyl group and Hex does a hexyl group, respectively.

Table 1

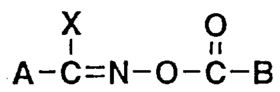

| A | X | B |
|---|---|---|
| A 1 | C 1 | B 1 |
| A 1 | C 1 | B 2 |
| A 1 | C 1 | B 3 |
| A 1 | C 1 | B 4 |
| A 1 | C 1 | B 5 |

Table 1 (continued)

| A | X | B |
|---|---|---|
| A 1 | C 1 | B 1 5 |
| A 1 | C 1 | B 1 6 |
| A 1 | C 1 | B 1 7 |
| A 1 | C 1 | B 2 5 |
| A 1 | C 1 | B 3 5 |
| A 2 | C 1 | B 1 |
| A 2 | C 1 | B 2 |
| A 2 | C 1 | B 3 |
| A 2 | C 1 | B 1 6 |
| A 2 | C 1 | B 1 7 |
| A 2 | C 1 | B 2 5 |
| A 2 | C 1 | B 2 7 |
| A 2 | C 1 | B 3 5 |
| A 3 | C 1 | B 1 |
| A 3 | C 1 | B 2 |
| A 3 | C 1 | B 3 |
| A 3 | C 1 | B 1 6 |
| A 3 | C 1 | B 1 7 |
| A 3 | C 1 | B 2 5 |
| A 3 | C 1 | B 2 7 |

Table 1 (continued)

| A | X | B |
|---|---|---|
| A 3 | C 1 | B 3 5 |
| A 4 | C 1 | B 1 |
| A 4 | C 1 | B 2 |
| A 4 | C 1 | B 3 |
| A 4 | C 1 | B 1 6 |
| A 4 | C 1 | B 1 7 |
| A 4 | C 1 | B 2 5 |
| A 4 | C 1 | B 2 7 |
| A 4 | C 1 | B 3 5 |
| A 5 | C 1 | B 1 |
| A 5 | C 1 | B 2 |
| A 5 | C 1 | B 3 |
| A 5 | C 1 | B 1 6 |
| A 5 | C 1 | B 1 7 |
| A 5 | C 1 | B 2 5 |
| A 5 | C 1 | B 2 7 |
| A 5 | C 1 | B 3 5 |
| A 6 | C 1 | B 1 |
| A 6 | C 1 | B 1 7 |
| A 6 | C 1 | B 2 5 |

Table 1 (continued)

| A | X | B |
|---|---|---|
| A 6 | C 1 | B 3 5 |
| A 7 | C 1 | B 1 |
| A 7 | C 1 | B 1 7 |
| A 7 | C 1 | B 2 5 |
| A 7 | C 1 | B 3 5 |
| A 8 | C 1 | B 1 |
| A 8 | C 1 | B 1 4 |
| A 8 | C 1 | B 2 5 |
| A 8 | C 1 | B 3 5 |
| A 9 | C 1 | B 1 |
| A 9 | C 1 | B 2 |
| A 9 | C 1 | B 3 |
| A 9 | C 1 | B 4 |
| A 9 | C 1 | B 5 |
| A 9 | C 1 | B 6 |
| A 9 | C 1 | B 7 |
| A 9 | C 1 | B 8 |
| A 9 | C 1 | B 9 |
| A 9 | C 1 | B 1 0 |
| A 9 | C 1 | B 1 1 |
| A 9 | C 1 | B 1 2 |
| A 9 | C 1 | B 1 3 |

Table 1 (continued)

| A | X | B |
|---|---|---|
| A 9 | C 1 | B 1 4 |
| A 9 | C 1 | B 1 5 |
| A 9 | C 1 | B 1 6 |
| A 9 | C 1 | B 1 7 |
| A 9 | C 1 | B 1 8 |
| A 9 | C 1 | B 1 9 |
| A 9 | C 1 | B 2 0 |
| A 9 | C 1 | B 2 1 |
| A 9 | C 1 | B 2 2 |
| A 9 | C 1 | B 2 3 |
| A 9 | C 1 | B 2 4 |
| A 9 | C 1 | B 2 5 |
| A 9 | C 1 | B 2 6 |
| A 9 | C 1 | B 2 7 |
| A 9 | C 1 | B 2 8 |
| A 9 | C 1 | B 2 9 |
| A 9 | C 1 | B 3 0 |
| A 9 | C 1 | B 3 1 |
| A 9 | C 1 | B 3 2 |
| A 9 | C 1 | B 3 3 |
| A 9 | C 1 | B 3 4 |
| A 9 | C 1 | B 3 5 |

Table 1 (continued)

| A | X | B |
|---|---|---|
| A 9 | C 1 | B 3 6 |
| A 9 | C 1 | B 3 7 |
| A 9 | C 1 | B 3 8 |
| A 9 | C 1 | B 3 9 |
| A 9 | C 1 | B 4 0 |
| A 9 | C 1 | B 4 1 |
| A 9 | C 1 | B 4 2 |
| A 9 | C 1 | B 4 3 |
| A 9 | C 1 | B 4 4 |
| A 9 | C 1 | B 4 5 |
| A 9 | C 1 | B 4 6 |
| A 9 | C 1 | B 4 7 |
| A 1 0 | C 1 | B 1 |
| A 1 0 | C 1 | B 2 |
| A 1 0 | C 1 | B 1 0 |
| A 1 0 | C 1 | B 1 3 |
| A 1 0 | C 1 | B 1 4 |
| A 1 0 | C 1 | B 1 7 |
| A 1 0 | C 1 | B 2 1 |
| A 1 0 | C 1 | B 2 2 |
| A 1 0 | C 1 | B 2 5 |
| A 1 0 | C 1 | B 3 5 |

Table 1 (continued)

| A | X | B |
|---|---|---|
| A11 | C1 | B1 |
| A11 | C1 | B2 |
| A11 | C1 | B8 |
| A11 | C1 | B10 |
| A11 | C1 | B13 |
| A11 | C1 | B14 |
| A11 | C1 | B17 |
| A11 | C1 | B21 |
| A11 | C1 | B22 |
| A11 | C1 | B25 |
| A11 | C1 | B35 |
| A12 | C1 | B1 |
| A12 | C1 | B2 |
| A12 | C1 | B8 |
| A12 | C1 | B10 |
| A12 | C1 | B13 |
| A12 | C1 | B14 |
| A12 | C1 | B17 |
| A12 | C1 | B21 |
| A12 | C1 | B22 |
| A12 | C1 | B25 |
| A12 | C1 | B35 |

Table 1 (continued)

| A | X | B |
|---|---|---|
| A 1 3 | C 1 | B 1 |
| A 1 3 | C 1 | B 2 |
| A 1 3 | C 1 | B 3 |
| A 1 3 | C 1 | B 1 0 |
| A 1 3 | C 1 | B 1 3 |
| A 1 3 | C 1 | B 1 4 |
| A 1 3 | C 1 | B 1 7 |
| A 1 3 | C 1 | B 2 1 |
| A 1 3 | C 1 | B 2 2 |
| A 1 3 | C 1 | B 2 5 |
| A 1 3 | C 1 | B 3 5 |
| A 1 4 | C 1 | B 1 |
| A 1 4 | C 1 | B 2 |
| A 1 4 | C 1 | B 3 |
| A 1 4 | C 1 | B 1 0 |
| A 1 4 | C 1 | B 1 3 |
| A 1 4 | C 1 | B 1 4 |
| A 1 4 | C 1 | B 1 7 |
| A 1 4 | C 1 | B 2 1 |
| A 1 4 | C 1 | B 2 2 |
| A 1 4 | C 1 | B 2 5 |
| A 1 4 | C 1 | B 3 5 |

Table 1 (continued)

| A | X | B |
|---|---|---|
| A15 | C1 | B1 |
| A15 | C1 | B2 |
| A15 | C1 | B3 |
| A15 | C1 | B10 |
| A15 | C1 | B13 |
| A15 | C1 | B14 |
| A15 | C1 | B17 |
| A15 | C1 | B21 |
| A15 | C1 | B22 |
| A15 | C1 | B25 |
| A15 | C1 | B35 |
| A16 | C1 | B1 |
| A16 | C1 | B2 |
| A16 | C1 | B3 |
| A16 | C1 | B10 |
| A16 | C1 | B13 |
| A16 | C1 | B14 |
| A16 | C1 | B17 |
| A16 | C1 | B21 |
| A16 | C1 | B22 |
| A16 | C1 | B25 |
| A16 | C1 | B35 |

Table 1 (continued)

| A | X | B |
|---|---|---|
| A17 | C1 | B1 |
| A17 | C1 | B2 |
| A17 | C1 | B3 |
| A17 | C1 | B10 |
| A17 | C1 | B13 |
| A17 | C1 | B14 |
| A17 | C1 | B17 |
| A17 | C1 | B21 |
| A17 | C1 | B22 |
| A17 | C1 | B25 |
| A17 | C1 | B35 |
| A18 | C1 | B1 |
| A18 | C1 | B2 |
| A18 | C1 | B3 |
| A18 | C1 | B10 |
| A18 | C1 | B13 |
| A18 | C1 | B14 |
| A18 | C1 | B17 |
| A18 | C1 | B21 |
| A18 | C1 | B22 |
| A18 | C1 | B25 |
| A18 | C1 | B35 |

Table 1 (continued)

| A | X | B |
|---|---|---|
| A 1 9 | C 1 | B 1 |
| A 1 9 | C 1 | B 2 |
| A 1 9 | C 1 | B 3 |
| A 1 9 | C 1 | B 1 0 |
| A 1 9 | C 1 | B 1 3 |
| A 1 9 | C 1 | B 1 4 |
| A 1 9 | C 1 | B 1 7 |
| A 1 9 | C 1 | B 2 1 |
| A 1 9 | C 1 | B 2 2 |
| A 1 9 | C 1 | B 2 5 |
| A 1 9 | C 1 | B 3 5 |
| A 2 0 | C 1 | B 1 |
| A 2 0 | C 1 | B 2 |
| A 2 0 | C 1 | B 3 |
| A 2 0 | C 1 | B 1 0 |
| A 2 0 | C 1 | B 1 3 |
| A 2 0 | C 1 | B 1 4 |
| A 2 0 | C 1 | B 1 7 |
| A 2 0 | C 1 | B 2 1 |
| A 2 0 | C 1 | B 2 2 |
| A 2 0 | C 1 | B 2 5 |
| A 2 0 | C 1 | B 3 5 |

Table 1 (continued)

| A | X | B |
|---|---|---|
| A 2 1 | C 1 | B 1 |
| A 2 1 | C 1 | B 2 |
| A 2 1 | C 1 | B 3 |
| A 2 1 | C 1 | B 4 |
| A 2 1 | C 1 | B 5 |
| A 2 1 | C 1 | B 6 |
| A 2 1 | C 1 | B 7 |
| A 2 1 | C 1 | B 8 |
| A 2 1 | C 1 | B 9 |
| A 2 1 | C 1 | B 1 0 |
| A 2 1 | C 1 | B 1 1 |
| A 2 1 | C 1 | B 1 2 |
| A 2 1 | C 1 | B 1 3 |
| A 2 1 | C 1 | B 1 4 |
| A 2 1 | C 1 | B 1 5 |
| A 2 1 | C 1 | B 1 6 |
| A 2 1 | C 1 | B 1 7 |
| A 2 1 | C 1 | B 1 8 |
| A 2 1 | C 1 | B 1 9 |
| A 2 1 | C 1 | B 2 0 |
| A 2 1 | C 1 | B 2 1 |
| A 2 1 | C 1 | B 2 2 |

Table 1 (continued)

| A | X | B |
|---|---|---|
| A 2 1 | C 1 | B 2 3 |
| A 2 1 | C 1 | B 2 4 |
| A 2 1 | C 1 | B 2 5 |
| A 2 1 | C 1 | B 2 6 |
| A 2 1 | C 1 | B 2 7 |
| A 2 1 | C 1 | B 2 8 |
| A 2 1 | C 1 | B 2 9 |
| A 2 1 | C 1 | B 3 0 |
| A 2 1 | C 1 | B 3 1 |
| A 2 1 | C 1 | B 3 2 |
| A 2 1 | C 1 | B 3 3 |
| A 2 1 | C 1 | B 3 4 |
| A 2 1 | C 1 | B 3 5 |
| A 2 1 | C 1 | B 3 6 |
| A 2 1 | C 1 | B 3 7 |
| A 2 1 | C 1 | B 3 8 |
| A 2 1 | C 1 | B 3 9 |
| A 2 1 | C 1 | B 4 0 |
| A 2 1 | C 1 | B 4 1 |
| A 2 1 | C 1 | B 4 2 |
| A 2 1 | C 1 | B 4 3 |
| A 2 1 | C 1 | B 4 4 |

Table 1 (continued)

| A | X | B |
|---|---|---|
| A21 | C1 | B45 |
| A21 | C1 | B46 |
| A21 | C1 | B47 |
| A22 | C1 | B1 |
| A22 | C1 | B2 |
| A22 | C1 | B10 |
| A22 | C1 | B13 |
| A22 | C1 | B14 |
| A22 | C1 | B17 |
| A22 | C1 | B25 |
| A22 | C1 | B35 |
| A23 | C1 | B1 |
| A23 | C1 | B2 |
| A23 | C1 | B10 |
| A23 | C1 | B13 |
| A23 | C1 | B14 |
| A23 | C1 | B22 |
| A23 | C1 | B25 |
| A23 | C1 | B35 |
| A24 | C1 | B1 |
| A24 | C1 | B10 |
| A24 | C1 | B13 |

Table 1 (continued)

| A | X | B |
|---|---|---|
| A24 | C1 | B14 |
| A24 | C1 | B17 |
| A24 | C1 | B22 |
| A24 | C1 | B25 |
| A24 | C1 | B35 |
| A25 | C1 | B1 |
| A25 | C1 | B10 |
| A25 | C1 | B13 |
| A25 | C1 | B14 |
| A25 | C1 | B17 |
| A25 | C1 | B22 |
| A25 | C1 | B25 |
| A25 | C1 | B35 |
| A26 | C1 | B1 |
| A26 | C1 | B10 |
| A26 | C1 | B13 |
| A26 | C1 | B14 |
| A26 | C1 | B17 |
| A26 | C1 | B22 |
| A26 | C1 | B25 |
| A26 | C1 | B35 |
| A26 | C1 | B39 |

Table 1 (continued)

| A | X | B |
|---|---|---|
| A27 | C1 | B1 |
| A27 | C1 | B10 |
| A27 | C1 | B13 |
| A27 | C1 | B14 |
| A27 | C1 | B17 |
| A27 | C1 | B22 |
| A27 | C1 | B25 |
| A27 | C1 | B35 |
| A28 | C1 | B1 |
| A28 | C1 | B10 |
| A28 | C1 | B13 |
| A28 | C1 | B14 |
| A28 | C1 | B17 |
| A28 | C1 | B22 |
| A28 | C1 | B25 |
| A28 | C1 | B35 |
| A29 | C1 | B1 |
| A29 | C1 | B10 |
| A29 | C1 | B13 |
| A29 | C1 | B14 |
| A29 | C1 | B17 |
| A29 | C1 | B22 |

Table 1 (continued)

| A | X | B |
|---|---|---|
| A29 | C1 | B25 |
| A29 | C1 | B35 |
| A30 | C1 | B1 |
| A30 | C1 | B14 |
| A30 | C1 | B17 |
| A30 | C1 | B22 |
| A30 | C1 | B25 |
| A30 | C1 | B35 |
| A31 | C1 | B1 |
| A31 | C1 | B14 |
| A31 | C1 | B17 |
| A31 | C1 | B22 |
| A31 | C1 | B25 |
| A31 | C1 | B35 |
| A32 | C1 | B1 |
| A32 | C1 | B14 |
| A32 | C1 | B17 |
| A32 | C1 | B22 |
| A32 | C1 | B25 |
| A32 | C1 | B35 |
| A33 | C1 | B1 |
| A33 | C1 | B10 |

Table 1 (continued)

| A | X | B |
|---|---|---|
| A33 | C1 | B13 |
| A33 | C1 | B14 |
| A33 | C1 | B17 |
| A33 | C1 | B22 |
| A33 | C1 | B25 |
| A33 | C1 | B35 |
| A34 | C1 | B1 |
| A34 | C1 | B10 |
| A34 | C1 | B13 |
| A34 | C1 | B14 |
| A34 | C1 | B17 |
| A34 | C1 | B22 |
| A34 | C1 | B25 |
| A34 | C1 | B35 |
| A35 | C1 | B1 |
| A35 | C1 | B10 |
| A35 | C1 | B13 |
| A35 | C1 | B14 |
| A35 | C1 | B17 |
| A35 | C1 | B22 |
| A35 | C1 | B25 |
| A35 | C1 | B35 |

Table 1 (continued)

| A | X | B |
|---|---|---|
| A36 | C1 | B1 |
| A36 | C1 | B10 |
| A36 | C1 | B13 |
| A36 | C1 | B14 |
| A36 | C1 | B17 |
| A36 | C1 | B22 |
| A36 | C1 | B25 |
| A36 | C1 | B35 |
| A37 | C1 | B1 |
| A37 | C1 | B10 |
| A37 | C1 | B13 |
| A37 | C1 | B14 |
| A37 | C1 | B17 |
| A37 | C1 | B22 |
| A37 | C1 | B25 |
| A37 | C1 | B35 |
| A38 | C1 | B1 |
| A38 | C1 | B10 |
| A38 | C1 | B13 |
| A38 | C1 | B14 |
| A38 | C1 | B17 |
| A38 | C1 | B22 |

Table 1 (continued)

| A | X | B |
|---|---|---|
| A38 | C1 | B25 |
| A38 | C1 | B35 |
| A39 | C1 | B1 |
| A39 | C1 | B10 |
| A39 | C1 | B13 |
| A39 | C1 | B14 |
| A39 | C1 | B17 |
| A39 | C1 | B22 |
| A39 | C1 | B25 |
| A39 | C1 | B35 |
| A40 | C1 | B1 |
| A40 | C1 | B10 |
| A40 | C1 | B13 |
| A40 | C1 | B14 |
| A40 | C1 | B17 |
| A40 | C1 | B22 |
| A40 | C1 | B25 |
| A40 | C1 | B35 |
| A41 | C1 | B1 |
| A41 | C1 | B10 |
| A41 | C1 | B13 |
| A41 | C1 | B14 |

Table 1 (continued)

| A | X | B |
|---|---|---|
| A41 | Cl | B17 |
| A41 | Cl | B22 |
| A41 | Cl | B25 |
| A41 | Cl | B35 |
| A9 | Br | B1 |
| A9 | Br | B10 |
| A9 | Br | B13 |
| A9 | Br | B14 |
| A9 | Br | B17 |
| A9 | Br | B22 |
| A9 | Br | B25 |
| A9 | Br | B35 |
| A13 | Br | B1 |
| A13 | Br | B10 |
| A13 | Br | B13 |
| A13 | Br | B14 |
| A13 | Br | B17 |
| A13 | Br | B22 |
| A13 | Br | B25 |
| A13 | Br | B35 |
| A21 | Br | B1 |
| A21 | Br | B10 |

Table 1 (continued)

| A | X | B |
|---|---|---|
| A21 | Br | B13 |
| A21 | Br | B14 |
| A21 | Br | B17 |
| A21 | Br | B22 |
| A21 | Br | B25 |
| A21 | Br | B35 |
| A37 | Br | B1 |
| A37 | Br | B10 |
| A37 | Br | B13 |
| A37 | Br | B14 |
| A37 | Br | B17 |
| A37 | Br | B22 |
| A37 | Br | B25 |
| A37 | Br | B35 |
| A38 | Br | B1 |
| A38 | Br | B10 |
| A38 | Br | B13 |
| A38 | Br | B14 |
| A38 | Br | B17 |
| A38 | Br | B22 |
| A38 | Br | B25 |
| A38 | Br | B35 |

Table 1 (continued)

| A | X | B |
|---|---|---|
| A41 | Br | B1 |
| A41 | Br | B10 |
| A41 | Br | B13 |
| A41 | Br | B14 |
| A41 | Br | B17 |
| A41 | Br | B22 |
| A41 | Br | B25 |
| A41 | Br | B35 |
| A9 | F | B1 |
| A9 | F | B10 |
| A9 | F | B13 |
| A9 | F | B14 |
| A9 | F | B17 |
| A9 | F | B22 |
| A9 | F | B25 |
| A9 | F | B35 |
| A13 | F | B1 |
| A13 | F | B10 |
| A13 | F | B13 |
| A13 | F | B14 |
| A13 | F | B17 |
| A13 | F | B22 |

Table 1 (continued)

| A | X | B |
|---|---|---|
| A 1 3 | F | B 2 5 |
| A 1 3 | F | B 3 5 |
| A 2 1 | F | B 1 |
| A 2 1 | F | B 1 0 |
| A 2 1 | F | B 1 3 |
| A 2 1 | F | B 1 4 |
| A 2 1 | F | B 1 7 |
| A 2 1 | F | B 2 2 |
| A 2 1 | F | B 2 5 |
| A 2 1 | F | B 3 5 |
| A 3 7 | F | B 1 |
| A 3 7 | F | B 1 0 |
| A 3 7 | F | B 1 3 |
| A 3 7 | F | B 1 4 |
| A 3 7 | F | B 1 7 |
| A 3 7 | F | B 2 2 |
| A 3 7 | F | B 2 5 |
| A 3 7 | F | B 3 5 |
| A 3 8 | F | B 1 |
| A 3 8 | F | B 1 0 |
| A 3 8 | F | B 1 3 |
| A 3 8 | F | B 1 4 |

Table 1 (continued)

| A | X | B |
|---|---|---|
| A38 | F | B17 |
| A38 | F | B22 |
| A38 | F | B25 |
| A38 | F | B35 |
| A41 | F | B1 |
| A41 | F | B10 |
| A41 | F | B13 |
| A41 | F | B14 |
| A41 | F | B17 |
| A41 | F | B22 |
| A41 | F | B25 |
| A41 | F | B35 |
| A9 | $SCH_3$ | B1 |
| A9 | $SCH_3$ | B10 |
| A9 | $SCH_3$ | B13 |
| A9 | $SCH_3$ | B14 |
| A9 | $SCH_3$ | B17 |
| A9 | $SCH_3$ | B22 |
| A9 | $SCH_3$ | B25 |
| A9 | $SCH_3$ | B35 |
| A9 | $SO_2CH_3$ | B1 |
| A9 | $SO_2CH_3$ | B10 |

Table 1 (continued)

| A | X | B |
|---|---|---|
| A 9 | $SO_2CH_3$ | B 1 3 |
| A 9 | $SO_2CH_3$ | B 1 4 |
| A 9 | $SO_2CH_3$ | B 1 7 |
| A 9 | $SO_2CH_3$ | B 2 2 |
| A 9 | $SO_2CH_3$ | B 2 5 |
| A 9 | $SO_2CH_3$ | B 3 5 |
| A 9 | SPh | B 1 |
| A 9 | SPh | B 1 0 |
| A 9 | SPh | B 1 3 |
| A 9 | SPh | B 1 4 |
| A 9 | SPh | B 1 7 |
| A 9 | SPh | B 2 2 |
| A 9 | SPh | B 2 5 |
| A 9 | SPh | B 3 5 |
| A 9 | $SO_2Ph$ | B 1 |
| A 9 | $SO_2Ph$ | B 1 0 |
| A 9 | $SO_2Ph$ | B 1 3 |
| A 9 | $SO_2Ph$ | B 1 4 |
| A 9 | $SO_2Ph$ | B 1 7 |
| A 9 | $SO_2Ph$ | B 2 2 |
| A 9 | $SO_2Ph$ | B 2 5 |
| A 9 | $SO_2Ph$ | B 3 5 |

Table 2

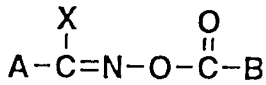

| A | X | B |
|---|---|---|
| A 9 | Cl | $CH_3$ |
| A 9 | Cl | Et |
| A 9 | Cl | Pr |
| A 9 | Cl | i-Pr |
| A 9 | Cl | Bu |
| A 9 | Cl | i-Bu |
| A 9 | Cl | t-Bu |
| A 9 | Cl | Pen |
| A 9 | Cl | Hex |
| A 9 | Cl | $CF_3$ |
| A 9 | Cl | $CH_2Cl$ |
| A 9 | Cl | $CCl_3$ |
| A 9 | Cl | $CHBrC(CH_3)$ |
| A 9 | Cl | $OCH_3$ |
| A 9 | Cl | OEt |
| A 9 | Cl | OPr |
| A 9 | Cl | OBu |
| A 9 | Cl | $NHCH_3$ |

Table 2 (continued)

| A | X | B |
|---|---|---|
| A 9 | C 1 | $N(CH_3)_2$ |
| A 9 | C 1 | NHEt |
| A 9 | C 1 | c-Pr |
| A 9 | C 1 | c-Pen |
| A 9 | C 1 | c-Hex |
| A 9 | C 1 | $CH=CHCH_3$ |
| A 9 | C 1 | $CH_2CH=CH_2$ |
| A 1 0 | C 1 | $CH_3$ |
| A 1 0 | C 1 | Et |
| A 1 0 | C 1 | $CF_3$ |
| A 1 0 | C 1 | $CHBrC(CH_3)_3$ |
| A 1 1 | C 1 | $CH_3$ |
| A 1 1 | C 1 | Et |
| A 1 1 | C 1 | $CF_3$ |
| A 1 1 | C 1 | $CHBrC(CH_3)_3$ |
| A 1 2 | C 1 | $CH_3$ |
| A 1 2 | C 1 | Et |
| A 1 2 | C 1 | $CF_3$ |
| A 1 2 | C 1 | $CHBrC(CH_3)_3$ |
| A 1 3 | C 1 | $CH_3$ |
| A 1 3 | C 1 | Et |
| A 1 3 | C 1 | Pr |

Table 2 (continued)

| A | X | B |
|---|---|---|
| A13 | Cl | i-Pr |
| A13 | Cl | Bu |
| A13 | Cl | $OCH_3$ |
| A13 | Cl | $NHCH_3$ |
| A13 | Cl | $N(CH_3)_2$ |
| A13 | Cl | NHPr |
| A13 | Cl | $CHBrC(CH_3)_3$ |
| A14 | Cl | $CH_3$ |
| A14 | Cl | Et |
| A14 | Cl | $CF_3$ |
| A15 | Cl | $CH_3$ |
| A15 | Cl | Et |
| A15 | Cl | $CHBrC(CH_3)_3$ |
| A16 | Cl | $CH_3$ |
| A16 | Cl | Et |
| A16 | Cl | $CF_3$ |
| A16 | Cl | $CHBrC(CH_3)_3$ |
| A17 | Cl | $CH_3$ |
| A17 | Cl | Et |
| A17 | Cl | $CF_3$ |
| A17 | Cl | $CHBrC(CH_3)_3$ |
| A18 | Cl | $CH_3$ |
| A18 | Cl | Et |

Table 2 (continued)

| A | X | B |
|---|---|---|
| A18 | Cl | $CF_3$ |
| A18 | Cl | $CHBrC(CH_3)_3$ |
| A21 | Cl | $CH_3$ |
| A21 | Cl | Et |
| A21 | Cl | Pr |
| A21 | Cl | i-Pr |
| A21 | Cl | Bu |
| A21 | Cl | i-Bu |
| A21 | Cl | t-Bu |
| A21 | Cl | Pen |
| A21 | Cl | Hex |
| A21 | Cl | $CF_3$ |
| A21 | Cl | $CH_2Cl$ |
| A21 | Cl | $CCl_3$ |
| A21 | Cl | $CHBrC(CH_3)_3$ |
| A21 | Cl | $OCH_3$ |
| A21 | Cl | OEt |
| A21 | Cl | OPr |
| A21 | Cl | OBu |
| A21 | Cl | $NHCH_3$ |
| A21 | Cl | $N(CH_3)_2$ |
| A21 | Cl | NHEt |
| A21 | Cl | c-Pr |

Table 2 (continued)

| A | X | B |
|---|---|---|
| A21 | Cl | c-Pen |
| A21 | Cl | c-Hex |
| A21 | Cl | $CH=CHCH_3$ |
| A21 | Cl | $CH_2CH=CH_2$ |
| A23 | Cl | $CH_3$ |
| A23 | Cl | Et |
| A23 | Cl | $CF_3$ |
| A23 | Cl | $CHBrC(CH_3)_3$ |
| A24 | Cl | $CH_3$ |
| A24 | Cl | Et |
| A24 | Cl | $CF_3$ |
| A26 | Cl | $CH_3$ |
| A26 | Cl | Et |
| A26 | Cl | $CF_3$ |
| A26 | Cl | $CHBrC(CH_3)_3$ |
| A27 | Cl | $CH_3$ |
| A27 | Cl | Et |
| A27 | Cl | $CF_3$ |
| A27 | Cl | $CHBrC(CH_3)_3$ |
| A33 | Cl | $CH_3$ |
| A33 | Cl | Et |
| A33 | Cl | $CF_3$ |
| A33 | Cl | $CHBrC(CH_3)_3$ |

Table 2 (continued)

| A | X | B |
|---|---|---|
| A37 | Cl | $CH_3$ |
| A37 | Cl | Et |
| A37 | Cl | Pr |
| A37 | Cl | i-Pr |
| A37 | Cl | Bu |
| A37 | Cl | i-Bu |
| A37 | Cl | t-Bu |
| A37 | Cl | Pen |
| A37 | Cl | Hex |
| A37 | Cl | $CF_3$ |
| A37 | Cl | $CH_2Cl$ |
| A37 | Cl | $CCl_3$ |
| A37 | Cl | $CHBrC(CH_3)_3$ |
| A37 | Cl | $OCH_3$ |
| A37 | Cl | OEt |
| A37 | Cl | OPr |
| A37 | Cl | OBu |
| A37 | Cl | $NHCH_3$ |
| A37 | Cl | $N(CH_3)_2$ |
| A37 | Cl | NHEt |
| A37 | Cl | c-Pr |
| A37 | Cl | c-Pen |
| A37 | Cl | c-Hex |

Table 2 (continued)

| A | X | B |
|---|---|---|
| A37 | Cl | $CH=CHCH_3$ |
| A37 | Cl | $CH_2CH=CH_2$ |
| A38 | Cl | $CH_3$ |
| A38 | Cl | Et |
| A38 | Cl | Pr |
| A38 | Cl | i-Pr |
| A38 | Cl | Bu |
| A38 | Cl | i-Bu |
| A38 | Cl | t-Bu |
| A38 | Cl | Pen |
| A38 | Cl | Hex |
| A38 | Cl | $CF_3$ |
| A38 | Cl | $CH_2Cl$ |
| A38 | Cl | $CCl_3$ |
| A38 | Cl | $CHBrC(CH_3)_3$ |
| A38 | Cl | $OCH_3$ |
| A38 | Cl | OEt |
| A38 | Cl | OPr |
| A38 | Cl | OBu |
| A38 | Cl | $NHCH_3$ |
| A38 | Cl | $N(CH_3)_2$ |
| A38 | Cl | NHEt |
| A38 | Cl | c-Pr |

Table 2 (continued)

| A | X | B |
|---|---|---|
| A 3 8 | C 1 | c-Pen |
| A 3 8 | C 1 | c-Hex |
| A 3 8 | C 1 | $CH=CHCH_3$ |
| A 3 8 | C 1 | $CH_2CH=CH_2$ |
| A 3 8 | C 1 | $CHBrC(CH_3)_3$ |
| A 3 9 | C 1 | $CH_3$ |
| A 3 9 | C 1 | Et |
| A 3 9 | C 1 | $CF_3$ |
| A 4 0 | C 1 | $CH_3$ |
| A 4 0 | C 1 | Et |
| A 4 0 | C 1 | $CF_3$ |
| A 4 1 | C 1 | $CH_3$ |
| A 4 1 | C 1 | Et |
| A 4 1 | C 1 | Pr |
| A 4 1 | C 1 | Bu |
| A 4 1 | C 1 | i-Bu |
| A 4 1 | C 1 | t-Bu |
| A 4 1 | C 1 | $CF_3$ |
| A 4 1 | C 1 | $CH_2Cl$ |
| A 4 1 | C 1 | $CCl_3$ |
| A 4 1 | C 1 | $CHBrC(CH_3)_3$ |
| A 4 1 | C 1 | $OCH_3$ |

Table 2 (continued)

| A | X | B |
|---|---|---|
| A41 | Cl | $NHCH_3$ |
| A41 | Cl | $N(CH_3)_2$ |
| A9 | Br | $CH_3$ |
| A13 | Br | $CH_3$ |
| A21 | Br | $CH_3$ |
| A37 | Br | $CH_3$ |
| A38 | Br | $CH_3$ |
| A41 | Br | $CH_3$ |
| A9 | $SCH_3$ | $CH_3$ |
| A13 | $SCH_3$ | $CH_3$ |
| A21 | $SCH_3$ | $CH_3$ |
| A37 | $SCH_3$ | $CH_3$ |
| A38 | $SCH_3$ | $CH_3$ |
| A41 | $SCH_3$ | $CH_3$ |
| A9 | $SO_2CH_3$ | $CH_3$ |
| A13 | $SO_2CH_3$ | $CH_3$ |
| A21 | $SO_2CH_3$ | $CH_3$ |
| A37 | $SO_2CH_3$ | $CH_3$ |
| A38 | $SO_2CH_3$ | $CH_3$ |
| A41 | $SO_2CH_3$ | $CH_3$ |

Table 3

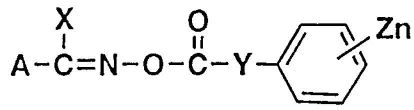

| A | X | Y | Zn |
|---|---|---|---|
| A 9 | C l | – | H |
| A 9 | C l | – | 4-F |
| A 9 | C l | – | 4-Cl |
| A 9 | C l | – | 4-$CH_3$ |
| A 9 | C l | – | 4-$OCH_3$ |
| A 9 | C l | – | 4-$CF_3$ |
| A 9 | C l | – | 4-CN |
| A 9 | C l | – | 4-$NO_2$ |
| A 9 | C l | – | 2-Cl |
| A 9 | C l | – | 3-Cl |
| A 9 | C l | – | 2-$CH_3$ |
| A 9 | C l | – | 2-$OCH_3$ |
| A 9 | C l | – | 2,4-$Cl_2$ |
| A 9 | C l | – | 3,4-$Cl_2$ |
| A 9 | C l | – | 3,5-$Cl_2$ |
| A 9 | C l | – | 2,4-$(CH_3)_2$ |
| A 9 | C l | – | 2,6-$F_2$ |
| A 9 | C l | – | 3-Cl-4-$CH_3$ |
| A 9 | C l | – | 3-$NO_2$-4-Cl |
| A 9 | C l | O | H |

Table 3 (continued)

| A | X | Y | Zn |
|---|---|---|---|
| A9 | Cl | O | 4-Cl |
| A9 | Cl | O | 4-$CH_3$ |
| A9 | Cl | NH | H |
| A9 | Cl | NH | 4-Cl |
| A9 | Cl | NH | 4-$CH_3$ |
| A10 | Cl | – | H |
| A10 | Cl | – | 4-Cl |
| A10 | Cl | – | 4-$CH_3$ |
| A10 | Cl | – | 4-$CF_3$ |
| A11 | Cl | – | H |
| A11 | Cl | – | 4-Cl |
| A11 | Cl | – | 4-$CH_3$ |
| A11 | Cl | – | 4-$CF_3$ |
| A12 | Cl | – | H |
| A12 | Cl | – | 4-Cl |
| A12 | Cl | – | 4-$CH_3$ |
| A12 | Cl | – | 4-$CF_3$ |
| A13 | Cl | – | H |
| A13 | Cl | – | 4-Cl |
| A13 | Cl | – | 4-$CH_3$ |
| A13 | Cl | – | 4-$CF_3$ |
| A14 | Cl | – | H |
| A14 | Cl | – | 4-Cl |

Table 3 (continued)

| A | X | Y | Zn |
|---|---|---|---|
| A14 | Cl | – | 4-$CH_3$ |
| A14 | Cl | – | 4-$CF_3$ |
| A15 | Cl | – | 4-Cl |
| A15 | Cl | – | 4-$CH_3$ |
| A16 | Cl | – | 4-Cl |
| A16 | Cl | – | 4-$CH_3$ |
| A17 | Cl | – | H |
| A17 | Cl | – | 4-Cl |
| A17 | Cl | – | 4-$CH_3$ |
| A17 | Cl | – | 4-$CF_3$ |
| A17 | Cl | – | 2,4-$Cl_2$ |
| A18 | Cl | – | H |
| A18 | Cl | – | 4-Cl |
| A18 | Cl | – | 4-$CH_3$ |
| A18 | Cl | – | 4-$CF_3$ |
| A19 | Cl | – | H |
| A19 | Cl | – | 4-Cl |
| A19 | Cl | – | 4-$CH_3$ |
| A19 | Cl | – | 4-$CF_3$ |
| A19 | Cl | – | 4-$NO_2$ |
| A20 | Cl | – | H |
| A20 | Cl | – | 4-Cl |
| A20 | Cl | – | 4-$CH_3$ |

Table 3 (continued)

| A | X | Y | Zn |
|---|---|---|---|
| A20 | Cl | – | 4-$CF_3$ |
| A21 | Cl | – | H |
| A21 | Cl | – | 4-Cl |
| A21 | Cl | – | 4-$CH_3$ |
| A21 | Cl | – | 4-$CF_3$ |
| A21 | Cl | – | 4-CN |
| A21 | Cl | – | 4-$NO_2$ |
| A21 | Cl | – | 2,4-$Cl_2$ |
| A21 | Cl | – | 3,4-$Cl_2$ |
| A21 | Cl | O | 4-Cl |
| A21 | Cl | NH | 4-Cl |
| A22 | Cl | – | H |
| A22 | Cl | – | 4-Cl |
| A22 | Cl | – | 4-$CH_3$ |
| A22 | Cl | – | 4-$CF_3$ |
| A23 | Cl | – | H |
| A23 | Cl | – | 4-Cl |
| A23 | Cl | – | 4-$CH_3$ |
| A23 | Cl | – | 4-$CF_3$ |
| A24 | Cl | – | H |
| A24 | Cl | – | 4-Cl |
| A24 | Cl | – | 4-$CH_3$ |
| A24 | Cl | – | 4-$CF_3$ |

Table 3 (continued)

| A | X | Y | Zn |
|---|---|---|---|
| A24 | Cl | – | 4-CN |
| A24 | Cl | – | 4-$NO_2$ |
| A25 | Cl | – | H |
| A25 | Cl | – | 4-Cl |
| A25 | Cl | – | 4-$CH_3$ |
| A25 | Cl | – | 4-$CF_3$ |
| A25 | Cl | – | 4-CN |
| A25 | Cl | – | 4-$NO_2$ |
| A26 | Cl | – | H |
| A26 | Cl | – | 4-Cl |
| A26 | Cl | – | 4-$CH_3$ |
| A26 | Cl | – | 4-$CF_3$ |
| A27 | Cl | – | H |
| A27 | Cl | – | 4-Cl |
| A27 | Cl | – | 4-$CH_3$ |
| A27 | Cl | – | 4-$CF_3$ |
| A28 | Cl | – | H |
| A28 | Cl | – | 4-Cl |
| A28 | Cl | – | 4-$CH_3$ |
| A28 | Cl | – | 4-$CF_3$ |
| A29 | Cl | – | H |
| A29 | Cl | – | 4-Cl |

Table 3 (continued)

| A | X | Y | Zn |
|---|---|---|---|
| A29 | Cl | – | 4-$CH_3$ |
| A29 | Cl | – | 4-$CF_3$ |
| A30 | Cl | – | H |
| A30 | Cl | – | 4-Cl |
| A30 | Cl | – | 4-$CH_3$ |
| A30 | Cl | – | 4-$CF_3$ |
| A31 | Cl | – | H |
| A31 | Cl | – | 4-Cl |
| A31 | Cl | – | 4-$CH_3$ |
| A31 | Cl | – | 4-$CF_3$ |
| A32 | Cl | – | H |
| A32 | Cl | – | 4-F |
| A32 | Cl | – | 4-Cl |
| A32 | Cl | – | 4-$CH_3$ |
| A32 | Cl | – | 4-$CF_3$ |
| A32 | Cl | – | 4-$OCH_3$ |
| A33 | Cl | – | H |
| A33 | Cl | – | 4-Cl |
| A33 | Cl | – | 4-$CH_3$ |
| A33 | Cl | – | 4-$CF_3$ |
| A34 | Cl | – | H |
| A34 | Cl | – | 4-Cl |

Table 3 (continued)

| A | X | Y | Zn |
|---|---|---|---|
| A34 | Cl | – | 4-$CH_3$ |
| A34 | Cl | – | 4-$CF_3$ |
| A35 | Cl | – | H |
| A35 | Cl | – | 4-Cl |
| A35 | Cl | – | 4-$CH_3$ |
| A35 | Cl | – | 4-$CF_3$ |
| A36 | Cl | – | H |
| A36 | Cl | – | 4-Cl |
| A36 | Cl | – | 4-$CH_3$ |
| A36 | Cl | – | 4-$CF_3$ |
| A37 | Cl | – | H |
| A37 | Cl | – | 4-Cl |
| A37 | Cl | – | 4-$CH_3$ |
| A37 | Cl | – | 4-$CF_3$ |
| A38 | Cl | – | H |
| A38 | Cl | – | 4-Cl |
| A38 | Cl | – | 4-$CH_3$ |
| A38 | Cl | – | 4-$CF_3$ |
| A39 | Cl | – | H |
| A39 | Cl | – | 4-Cl |
| A40 | Cl | – | H |
| A40 | Cl | – | 4-Cl |

Table 3 (continued)

| A | X | Y | Zn |
|---|---|---|---|
| A40 | Cl | – | 4-$CH_3$ |
| A40 | Cl | – | 4-$CF_3$ |
| A41 | Cl | – | H |
| A41 | Cl | – | 4-Cl |
| A41 | Cl | – | 4-$CH_3$ |
| A41 | Cl | – | 4-$CF_3$ |
| A41 | Cl | O | 4-Cl |
| A41 | Cl | O | 4-$CH_3$ |
| A41 | Cl | NH | 4-Cl |
| A41 | Cl | NH | 4-$CH_3$ |
| A9 | Br | – | H |
| A9 | Br | – | 4-Cl |
| A9 | Br | – | 4-$CH_3$ |
| A9 | Br | – | 4-$OCH_3$ |
| A9 | Br | – | 4-$CF_3$ |
| A9 | Br | O | H |
| A9 | Br | O | 4-Cl |
| A9 | Br | NH | H |
| A9 | Br | NH | 4-Cl |
| A21 | Br | – | H |
| A21 | Br | – | 4-Cl |
| A21 | Br | – | 4-$CH_3$ |

Table 3 (continued)

| A | X | Y | Zn |
|---|---|---|---|
| A21 | Br | – | $4-OCH_3$ |
| A21 | Br | – | $4-CF_3$ |
| A37 | Br | – | H |
| A37 | Br | – | 4-Cl |
| A37 | Br | – | $4-CH_3$ |
| A37 | Br | – | $4-OCH_3$ |
| A37 | Br | – | $4-CF_3$ |
| A38 | Br | – | H |
| A38 | Br | – | 4-Cl |
| A38 | Br | – | $4-CH_3$ |
| A38 | Br | – | $4-OCH_3$ |
| A38 | Br | – | $4-CF_3$ |
| A41 | Br | – | H |
| A41 | Br | – | 4-Cl |
| A41 | Br | – | $4-CH_3$ |
| A41 | Br | – | $4-OCH_3$ |
| A41 | Br | – | $4-CF_3$ |
| A9 | F | – | H |
| A9 | F | – | 4-Cl |
| A9 | F | – | $4-CH_3$ |
| A9 | F | – | $4-OCH_3$ |
| A9 | F | – | $4-CF_3$ |

Table 3 (continued)

| A | X | Y | Zn |
|---|---|---|---|
| A9 | F | O | 4-Cl |
| A9 | F | NH | 4-Cl |
| A9 | $SCH_3$ | – | H |
| A9 | $SCH_3$ | – | 4-Cl |
| A9 | $SCH_3$ | – | 4-$CH_3$ |
| A9 | $SCH_3$ | – | 4-$OCH_3$ |
| A9 | $SCH_3$ | – | 4-$CF_3$ |
| A9 | $SCH_3$ | O | 4-Cl |
| A9 | $SCH_3$ | NH | 4-Cl |
| A9 | $SO_2CH_3$ | – | H |
| A9 | $SO_2CH_3$ | – | 4-Cl |
| A9 | $SO_2CH_3$ | – | 4-$CH_3$ |
| A9 | $SO_2CH_3$ | – | 4-$OCH_3$ |
| A9 | $SO_2CH_3$ | – | 4-$CF_3$ |
| A9 | $SO_2CH_3$ | O | 4-Cl |
| A9 | $SO_2CH_3$ | NH | 4-Cl |
| A13 | $SCH_3$ | – | H |
| A13 | $SCH_3$ | – | 4-Cl |
| A13 | $SCH_3$ | – | 4-$CH_3$ |
| A13 | $SCH_3$ | – | 4-$OCH_3$ |
| A21 | $SCH_3$ | – | H |
| A21 | $SCH_3$ | – | 4-Cl |

Table 3 (continued)

| A | X | Y | Zn |
|---|---|---|---|
| A21 | $SCH_3$ | – | 4-$CH_3$ |
| A21 | $SCH_3$ | – | 4-$OCH_3$ |
| A37 | $SCH_3$ | – | H |
| A37 | $SCH_3$ | – | 4-Cl |
| A37 | $SCH_3$ | – | 4-$CH_3$ |
| A37 | $SCH_3$ | – | 4-$OCH_3$ |
| A38 | $SCH_3$ | – | H |
| A38 | $SCH_3$ | – | 4-Cl |
| A38 | $SCH_3$ | – | 4-$CH_3$ |
| A38 | $SCH_3$ | – | 4-$OCH_3$ |
| A41 | $SCH_3$ | – | H |
| A41 | $SCH_3$ | – | 4-Cl |
| A41 | $SCH_3$ | – | 4-$CH_3$ |
| A41 | $SCH_3$ | – | 4-$OCH_3$ |
| A41 | $SO_2CH_3$ | – | H |
| A41 | $SO_2CH_3$ | – | 4-Cl |
| A41 | $SO_2CH_3$ | – | 4-$CH_3$ |
| A41 | $SO_2CH_3$ | – | 4-$OCH_3$ |
| A9 | SPh | – | H |
| A9 | SPh | – | 4-Cl |
| A9 | SPh | – | 4-$CH_3$ |
| A9 | SPh | – | 4-$OCH_3$ |

Table 3 (continued)

| A | X | Y | Zn |
|---|---|---|---|
| A 9 | $SO_2Ph$ | – | H |
| A 9 | $SO_2Ph$ | – | 4-Cl |

In the above-mentioned Tables 1 to 3, A1 to A41 show the following chemical structures.

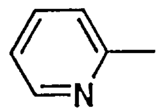

A1 ,

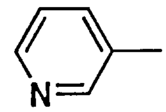

A2 ,

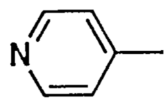

A3 ,

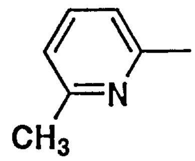

A4 ,

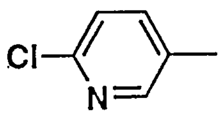

A5 ,

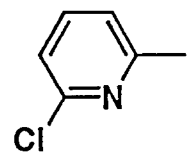

A6 ,

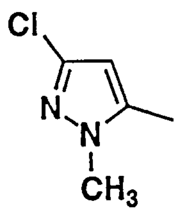

A7 ,

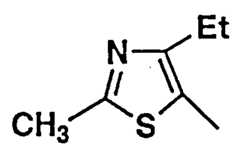

A8 ,

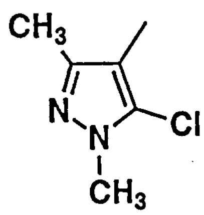

A9 ,

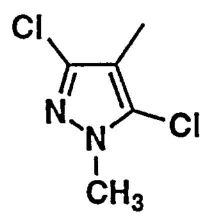

A10 ,

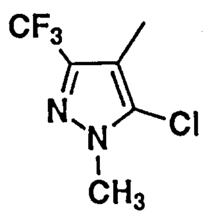

A11 ,

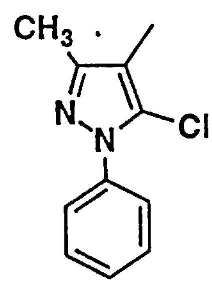

A12 ,

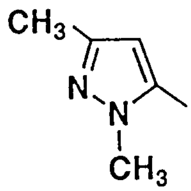 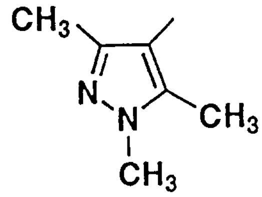

A13 , A14 ,

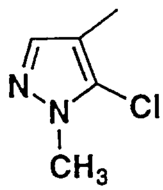 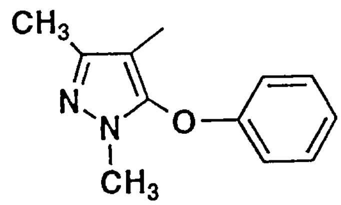

A15 , A16 ,

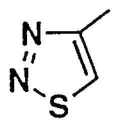 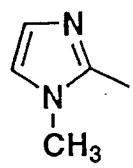

A17 , A18 ,

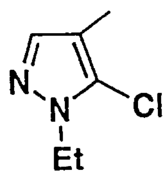 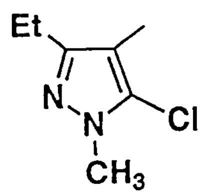

A19 , A20 ,

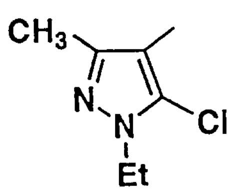 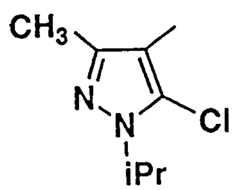

A21 , A22 ,

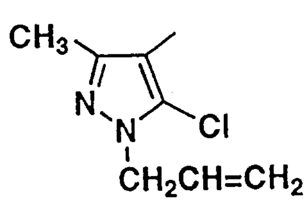 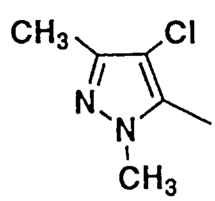

A23 , A24 ,

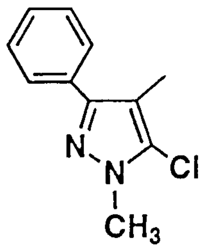 ,

A25

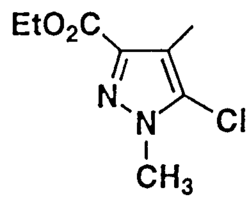 ,

A26

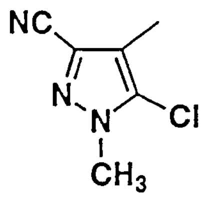 ,

A27

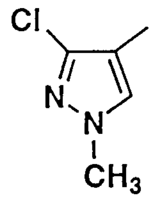 ,

A28

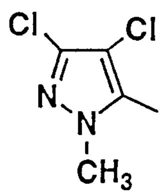 ,

A29

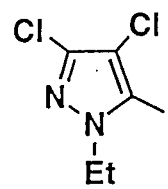 ,

A30

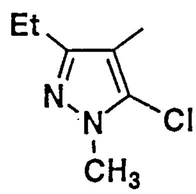 ,

A31

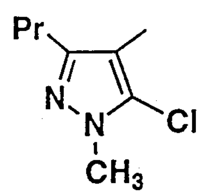 ,

A32

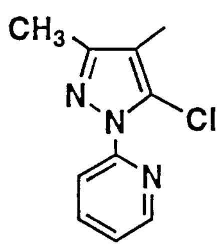 ,

A33

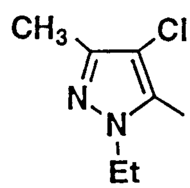 ,

A34

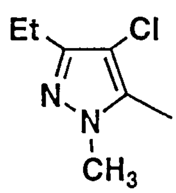 ,

A35

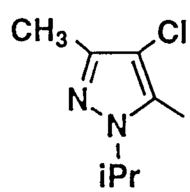 ,

A36

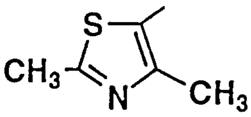

,

A37

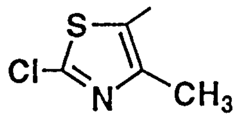

,

A38

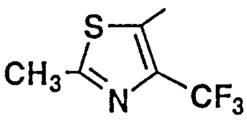

,

A39

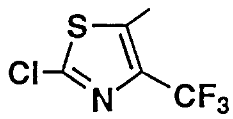

,

A40

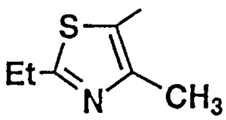

,

A41

In the above Tables 1 to 3, B1 to B47 show the following chemical structures.

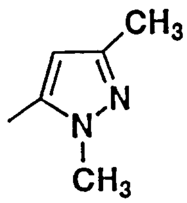

B1 ,

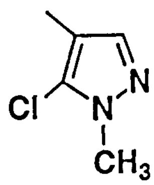

B2 ,

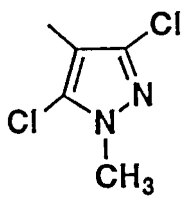

B3 ,

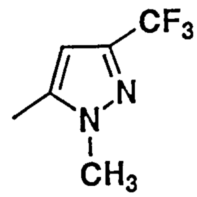

B4 ,

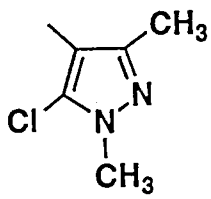

B5 ,

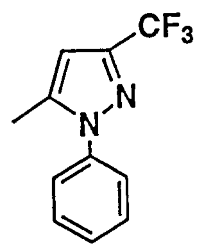

B6 ,

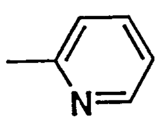

B7 ,

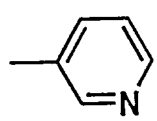

B8 ,

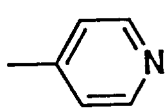

B9 ,

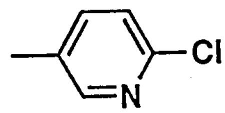

B10 ,

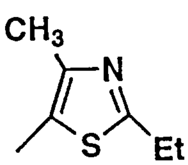

B11 ,

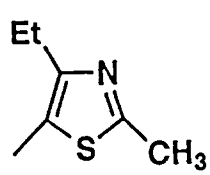

B12 ,

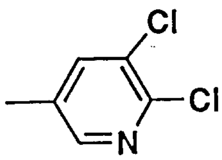

B13 ,

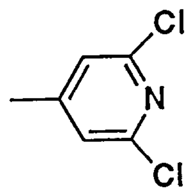

B14 ,

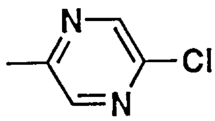

B15 ,

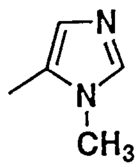

B16 ,

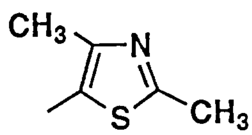

B17 ,

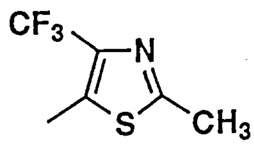

B18 ,

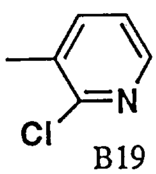

B19 ,

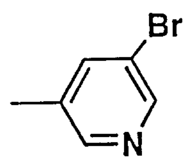

B20 ,

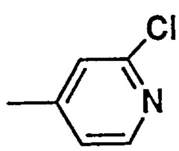

B21 ,

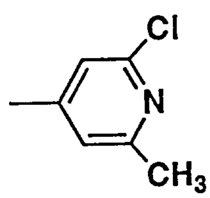

B22 ,

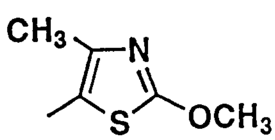

B23 ,

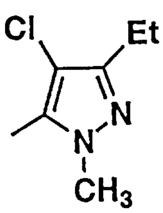

B24 ,

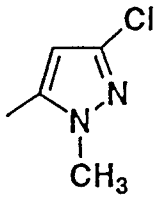
,
B25

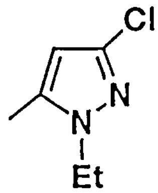
,
B26

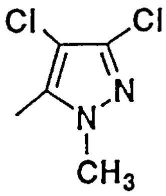
,
B27

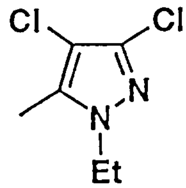
,
B28

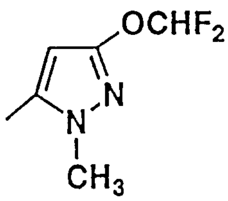
,
B29

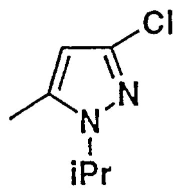
,
B30

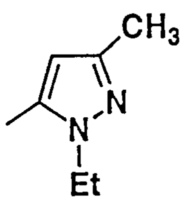
,
B31

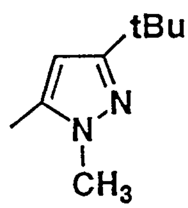
,
B32

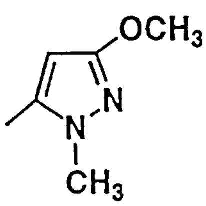
,
B33

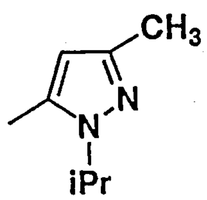
,
B34

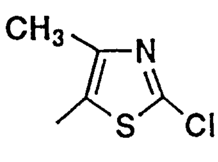
,
B35

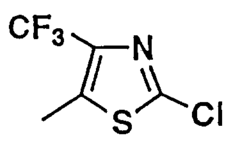
,
B36

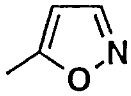

B37 ,

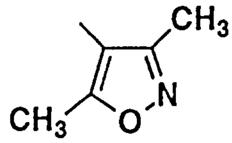

B38 ,

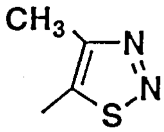

B39 ,

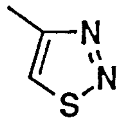

B40 ,

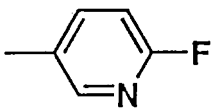

B41 ,

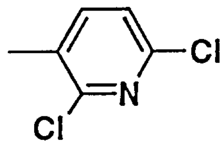

B42 ,

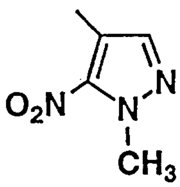

B43 ,

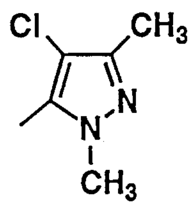

B44 ,

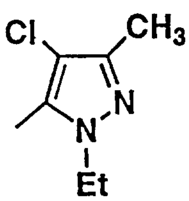

B45 ,

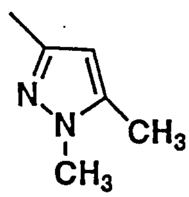

B46 ,

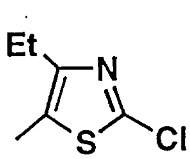

B47 ,

The process for producing the compound of the present invention will be described below.

(Process 1)

The compound of the present invention can be produced by reacting a compound of formula [3]

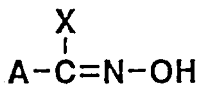

[3]

wherein A and X have the same meanings as defined above, with a compound of formula [4]

B-COL (4)

wherein B has the same meaning as defined above, and L represents a leaving group such as a halogen atom or the like.

(Process 2)

A process in the case of X = $S(O)nR^{24}$:

A compound of formula [7]

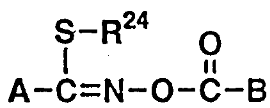

[7]

wherein A, B and $R^{24}$ have the same meanings defined above, can be producedby reacting a compound of formula [5]

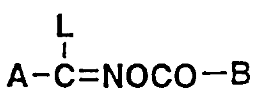

[5]

wherein A and B are as defined above, and L represents a leaving group such as a halogen atom or the like, with a compound of formula [6]

$R^{24}S$ (6)

wherein $R^{24}$ has the same meaning as defined above, and M represents an alkali metal atom such as Na or the like.

A compound of the present invention of formula [8]

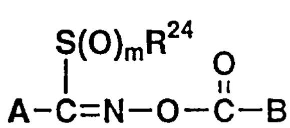

[8]

wherein A, B and $R^{24}$ have the same meanings as defined above, and m is 0 or an integer of 1 to 2, can be produced by oxidizing this compound of the formula [7] with an appropriate oxidizing agent (for example, hydrogen peroxide or mCPBA).

In Process 1, the reaction can be conducted using an appropriate inert solvent. The solvent includes, for example, aromatic hydrocarbons such as toluene, xylene, chlorobenzene and the like; halogenated hydrocarbons such as dichloroethane and the like; ethers such as diisopropyl ether, dioxane and the like; esters such as ethyl acetate and the like; nitriles such as acetonitrile and polar solvents such as dimethyl sulfoxide, dimethylformamide and the like.

Organic bases (pyridine, triethylamine and the like) or inorganic bases (potassium carbonate, sodium hydride and the like) may be added in accordance with the necessity.

With respect to the amount of the reagent used in the reaction, the amount of the compound of formula [4] is within the range of from 1 to 3 equivalents per equivalent of the compound of formula [3].

The above-mentioned reaction can be conducted at any reaction temperature. Generally, a temperature of from 0 to 200 °C or a reflux temperature of the solvent is preferable.

After the completion of the reaction, a usual post-treatment is carried out, whereby the final compound can be obtained.

In Process 2 as well, the reaction can be conducted using an appropriate inert solvent. The solvent used includes, for example, aromatic hydrocarbons such as toluene, xylene, chlorobenzene and the like; halogenated hydrocarbons such as dichloroethane and the like; ethers such as diisopropyl ether, dioxane and the like; alcohols such as methyl alcohol, ethyl alcohol and the like; polar solvents such as dimethyl sulfoxide, dimethylformamide and the like.

With respect to the amount of the reagent used in the reaction, the amount of the compound of formula [5] is within the range of from 1 to 3 equivalents per equivalent of the compound of formula [5].

The above-mentioned reaction can be conducted at any reaction temperature. Normally, a temperature of from 0 to 200 °C or a reflux temperature of the solvent is preferable.

Compounds of the present invention are effective for protecting plants from various plant diseases caused by, for example, Pyricularia oryzae, Cochliobolus miyabeanus, Rhizoctonia solani, Erysiphe graminis f. sp. hordei, f. sp. tritici, Pyrenophora graminea, Pyrenophora teres, Gibberella zeae, Puccinia striiformis, P. graminis, P. recondita, P. hordei, Typhula sp., Micronectriella nivais, Ustilago tritici, U. nuda, Pseudocercosporella herpotrichoides, Rhynchosporium secalis, Septoria tritici, Leptosphaeria nodorum, Diaporthe citri, Elsinoe fawcetti, Penicillium digitatum, P. italicum, Sclerotinia mali, Valsa mali, Podosphaera leucotricha, Alternaria mali, Venturia inaequalis, Venturia nashicola, Alternaria Kikuchiana, Gymnosporangium haraeanum, Sclerotinia cinerea, Cladosporium carpophilum, Phomopsis sp., Plasmopara viticola, Elsinoe ampelina, Glomerella cingulata, Uncinula necator, Phakopsora ampelopsidis, Gloeosporium kaki, Cercospora kaki, Mycosphaerella nawae, Pseudoperenospora cubensis, Colletotrichum lagenarium, Sphaerotheca fuliginea, Mycosphaerella melonis, Phytophthora infestans, Alternaria solani, Cladosporium fulvam, Phomopsis vexans, Erysiphe cichoracoarum, Alternaria japonica, Cerocosporella brassicae, Puccinia allii, Cerospora kikuchii, Elsinoe glycines, Diaporthe phaseololum, Colletotrichum lindemuthianum, Mycosphaerella personatum, Cercospora arachidicola, Erysiphe pisi, Alternaria solani, Sphaerotheca humuli, Exobasidium reticulatum, Elsinoe leucospila, Alternaria longipes, Erysiphe cichoracearum, Colletotrichum tabacum, Cercospora beticola, Diplocarpon rosae, Sphaerotheca pannosa, Septoria chrysanthemiidici, Puccinia horiana, Botrytis cinerea, Sclerotinia sclerotiorum.

Where compounds of the present invention are used as bactericides for agricultural and horticultural use, in general, they may be mixed with a suitable carrier, for example, a solid carrier such as clay, talc, bentonite, diatomaceous earth or the like, or a liquid carrier such as water, alcohols (e.g., methanol, ethanol, etc.), aromatic hydrocarbons (e.g., benzene, toluene, methylnaphthalene, etc.), chlorinated hydrocartons, ethers, ketones, esters (e.g., ethyl acetate, etc.), acid amides (e.g., dimethylformamide, etc.) or the like. If desired, they may be mixed with an emulsifier, a dispersing agent, a suspending agent, a penetrating agent, a spreader, a stabilizer and the like to be formed into various practical formulations of liquid formulation, emulsifiable concentrate, wettable powder, dust, granules, flowable or the like.

When the compounds of the present invention are used as an agricultural and horticultural bactericide, these compounds are applied by foliar application, soil treatment, seed disinfection or the like. Application methods which are generally used by thosed skilled in the art are also available.

If desired, they may also be combined with any other herbicides, various insecticides, fungicides, plant growth regulators, synergists and others, in forming them into formulations or in actually applying them onto plants.

The amount of the compound of the present invention to be applied to plants varies, depending upon the place, time, method, plant diseases, growth crops and other conditions. In general, the effective amount is suitably from 0.005 to 50 kg or so per hectare.

Next, some examples of fungicidal formulations containing the compound of the present invention as an active ingredient are shown below, which, however, are not limitative. In the following examples, "parts" are by weight.

Formulation Example 1: Emulsifiable Concentrate

| | |
|---|---|
| Compound of the invention | 20 parts |
| Methyl naphthalene | 55 parts |
| Cyclohexane | 20 parts |
| Sorpol 2680 (trade name, a mixture of a non-ionic surface-active agent and an anionic surface-active agent manufactured by Toho Kagaku Kogyo Kabushiki Kaisha) | 5 parts |

The above ingredients are uniformly mixed to form an emulsfiable concentrate. Before use, the emulsifiable concentrate is diluted to from 1/50 to 1/20000, and the diluted emulsifiable concentrate is applied to a crop field in an amount of from 0.005 to 50 kg, as the active ingredient, per hectare.

Formulation Example 2: Wettable Powder

| | |
|---|---|
| Compound of the invention | 25 parts |
| Zeeklite PFP (trade name, a mixture of kaolinite and sericite manufactured by Zeeklite Mining Industries Co., Ltd.) | 66 parts |
| Sorporl 5039 (trade name, an anionic surface-active agent manufactured by Toho Kagaku Kogyo Kabushiki Kaisha) | 4 parts |
| Carplex #80 (trade name, white carbon manufactured by Shionogi Seiyaku Kabushiki Kaisha) | 3 parts |
| Calcium lignin sulfonate | 2 parts |

The above ingredients are uniformly mixed and milled to form a wettable powder. Before use, the powder is diluted with water to from 1/50 to 1/20000, and the diluted liquid is applied to a crop field in an amount of from 0.005 to 50 kg, as the active ingredient, per hectare.

Formulation Example 3: Dust

| | |
|---|---|
| Compound of the invention | 3 parts |
| Carplex #80 (trade name, white carbon manufactured by Shionogi Seiyaku Kabushiki Kaisha) | 0.5parts |
| Clay | 95 parts |
| Diisopropyl phosphate | 1.5parts |

The above ingredients are uniformly mixed and milled to form dust. For use, this is applied to a crop field in an amount of from 0.005 to 50 kg, as the active ingredient, per hectare.

Formulation Example 4: Granules

| | |
|---|---|
| Compound of the invention | 5 parts |
| Bentonite | 30 parts |
| Talc | 64 parts |
| Calcium lignin sulfonate | 1 part |

The above ingredients are uniformly mixed and milled, and a small amount of water is added thereto and mixed with stirring. The mixture is granulated through an granulating extruder and dried to form granules. For use, the granules are applied to a crop field in an amount of from 0.005 to 50 kg, as the active ingredient, per hectare.

Formulation Example 5: Flowable

| | |
|---|---|
| Compound of the invention | 25 parts |
| Sorporl 3353 (trade name, a nonionic surface-active agent manufactured by Toho Kagaku Kogyo Kabushiki Kaisha) | 5 parts |
| Lunox 1000C (trade name, an anionic surface-active agent manufactured by Toho Kagaku Kogyo Kabushiki Kaisha) | 0.5 part |
| Xanthan gum (natural high-molecular gum) | 0.2 part |
| Sodium benzoate | 0.4 part |
| Propylene glycol | 10 parts |
| Water | 58.9 parts |

The above ingredients except the active ingredient are uniformly mixed, and the compound of the invention is added thereto and well stirred. The resulting mixture is then wet-milled in a sand mill to obtain a flowable. Before use, this is diluted to from 1/50 to 1/2000, and the diluted liquid is applied to a crop field in an amount of from 0.005 to 50 kg, as the active ingredient, per hectare.

The present invention will be illustrated specifically by referring to the following Examples. However, the present invention is not limited to these Examples.

Best Mode of Carrying Out the Invention

Example 1

Synthesis of Compound No. 1-1 of the Present Invention

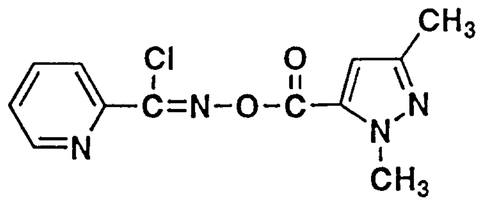

2.23 g of triethylamine (22 mmols) was dropwise added to a suspension of 1.93 g of 2-(α-chloroformaldoxime)-pyridinium hydrochloride (10 mmols) in 100 ml of methylene chloride at 5 °C . After the mixture was stirred as such for 15 minutes, 1.59 g of 1,3-dimethylpyrazole-5-carbonyl chloride (10 mmols) were dropwise added thereto. The temperature was gradually elevated to room temperature, and the mixture was then stirred for 30 minutes.

Subsequently, 100 ml of water were added thereto, and the resulting mixture was extracted with methylene chloride, and dried over anhydrous sodium sulfate. The solvent was then distilled off under reduced pressure. The thus-obtained crystals were washed with a mixed solvent of diisopropyl ether and methanol (5 : 1) to obtain 1.2 g of the intended O-(1,3-dimethylpyrazole-5-carbonyl)-2-(α-chloroformaldoxime) pyridine. melting point: 143 to 144 °C

Example 2

Synthesis of Compound No. 1-5 of the Present Invention

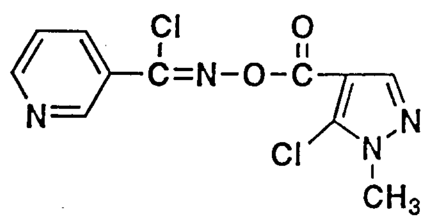

2.23 g of triethylamine (22 mmols) was dropwise added to a suspension of 1.93 g of 3-(α-chloroformaldoxime)-pyridinium hydrochloride (10 mmols) in 100 ml of methylene chloride at 5 °C . After the mixture was stirred as such for 15 minutes, a solution of 1.79 g of 1-methyl-5-chloropyrazole-4-carbonyl chloride (10 mmols) in 5 ml of methylene chloride was dropwise added thereto. The temperature was gradually elevated to room temperature, and the mixture was then stirred for 1 hour. Thereafter, 100 ml of water were added thereto, and the resulting mixture was extracted with methylene chloride, and dried over anhydrous sodium sulfate. The solvent was then distilled off under reduced pressure. The thus-obtained crystals were washed with a mixed solvent of isopropyl ether and methanol (5 : 1) to obtain 0.97 g of the intended 0-(1-methyl-5-chloropyrazole-4-carbonyl)-3-(α-chloroformaldoxime)-pyridine.

melting point: 121 to 123 °C

Example 3

Synthesis of Compound No. 1-9 of the Present Invention

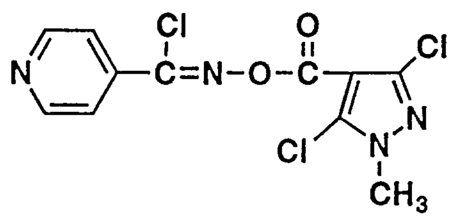

2.23 g of triethylamine (22 mmols) was dropwise added to a suspension of 1.93 g of 4-(α-chloroformaldoxime)-pyridinium hydrochloride (10 mmols) in 100 ml of methylene chloride at 5 °C . After the mixture was stirred as such for 30 minutes, a solution of 2.14 g of 1-methyl-3,5-dichloropyrazole-4-carbonyl chloride (10 mmols) in 5 ml of methylene chloride was dropwise added thereto. The temperature was gradually elevated to room temperature, and the mixture was then stirred for 12 hours. Thereafter, 100 ml of water were added thereto, and the resulting mixture was extracted with methylene chloride, and dried over anhydrous sodium sulfate. The solvent was then distilled off under reduced pressure. The thus-obtained crystals were washed with methanol to obtain 1.6 g of the intended O-(1-methyl-3,5-dichloropyrazole-4-carbonyl)-4-(α-chloroformaldoxime)-pyridine.

melting point: 165 to 168 °C

Example 4

Synthesis of Compound No. 1-18 of the Present Invention

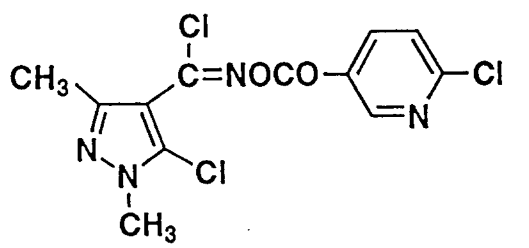

(1) Synthesis of 5-chloro-4-(α-chloroformaldoxime)-1,3-dimethylpyrazole hydrochloride:

1.74 g of 5-chloro-1,3-dimethylpyrazole-4-formaldoxime (10 mmols) was suspended in 50 ml of dichloromethane, and a chlorine gas was blown at a temperature of from -15 °C to -5 °C . The reaction mixture was heated to room temperature, and stirred as such for 3 hours. The crystals precipitated were then collected by filtration, washed with dichloromethane and dried to give 1.65 g of 5-chloro-4-(α-chloroformaldoxime)-1,3-dimethylpyrazole hydrochloride. melting point: 140 to 143 °C .

(2) Synthesis of Compound No. 1-18 of the Present Invention

1.6 g of 5-chloro-4-(α-chloroformaldoxime)-1,3-dimethylpyrazole hydrochloride (6.54 mmols) and 1.15 g of 6-chloronicotinoyl chloride (6.54 mmols) were suspended in 70 ml of dichloromethane, and 1.33 g of triethylamine (13.1 mmols) were dropwise added thereto at 5 °C while cooling the suspension with ice. Subsequently, the mixture was heated to room temperature, and stirred as such for 30 minutes. A catalytic amount of 4-dimethylaminopyridine was further added thereto, and the mixture was further stirred for 1 hour.

50 ml of dichloromethane and 100 ml of water were added to the reaction mxiture. The dichloromethane layer was dried over anhydrous sodium sulfate, and the solvent was then distilled off under reduced pressure. The thus-obtained

crystals were washed with isopropyl ether, and collected by filtration to obtain 0.7 g of the intended 0-(6-chloronicotinoyl-5-chloro-4-(α-chloroformaldoxime)-1,3-ethyl-pyrazole as pale yellowish brown crystals. melting point: 123.0 to 126.0 °C

Example 5

Synthesis of Compound No. 1-20 of the Present Invention

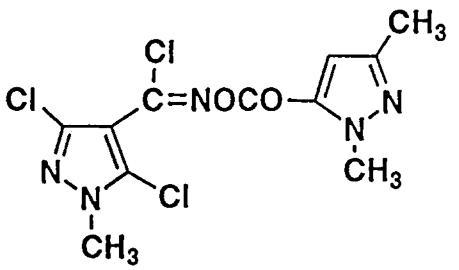

(1) Synthesis of 4-(α-chloroformaldoxime)-3,5-dichloro-1-methylpyrazole:

2 g of 3,5-dichloro-4-formaldoxime-1-methyl-prazole (10.3 mmols) were suspended in 50 ml of dichloromethane, and a chlorine gas was blown at a temperature of from -15 °C to 0 °C for 20 minutes. The mixture was then heated to room temperature, and stirred as such for 30 minutes. The solvent was distilled off from the reaction mixture under reduced pressure. The thus-obtained crystals were washed with hexane, and collected by filtration to obtain 1.2 g of the intended 4-(α-chloroformaldoxime)-3,5-dichloro-1-methyl-pyrazole as white crystals. melting point: 134 to 135 °C

(2) Synthesis of Invention Compound No. 1-20:

0.5 g (2.19 mols) of 4-(α-chloroformaldoxime)-3,5-dichloro-1-methylpyrazole and 0.35 g (2.19 mmols) of 1,3-dimethylpyrazole-5-carbonyl chloride were suspended in 40 ml of dichloromethane, and 0.23 g of triethylamine (2.3 mmols) were dropwise added thereto while stirring the suspension. Further, a catalytic amount of 4-dimethylaminopyridine was added thereto. After the resulting mixture was stirred for 2 hours, 50 ml of dichloromethane and 50 ml of water were added to the reaction mixture. The dichloromethane layer was dried over anhydrous sodium sulfate, and the solvent was then distilled off under reduced pressure. The thus-obtained crystals were washed with isopropyl ether, and collected by filtration to obtain 0.4 g of the intended O-(1,3-dimethylpyrazole-5-carbonyl)-4-(α-chloroformaldoxime)-3,5-dichloro-1-methylpyrazole as pale yellowish brown crystals. melting point: 121 to 122 °C

Example 6

Synthesis of Compound No. 1-25 of the Present Invention

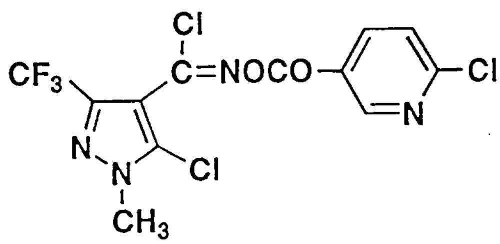

(1) Synthesis of 4-(α-chloroformaldoxime)-5-chloro-1-methyl-3-trifluoromethylpyrazole:

4.0 g (17.6 mmols) of 4-formaldoxime-5-chloro-1-methyl-3-trifluoromethylpyrazole were dissolved in 50 ml of dichloromethane, and a chlorine gas was blown in small portions at 3 °C over a period of 50 minutes while cooling the solution with ice. The mixture was stirred as such for 1 hour, and the temperature was then elevated to room temperature. The solvent was distilled off from the reaction mixture under reduced pressure. The thus-obtained crystals were washed with hexane, and collected by filtration to obtain 3.38 g of the intended 4-(α-chloroformaldoxime)-5-chloro-1-methyl-3-trifluoromethylpyrazole. melting point: 99 to 101 °C

(2) Synthesis of Compound No. 1-25 of the Present Invention

0.5 g of 4-(α-chloroformaldoxime)-5-chloro-1-methyl-3-trifluoromethylpyrazole (1.91 mmols) and 0.34 g of 6-chloronicotinoyl chloride (1.91 mmols) were suspended in 40 ml of dichloromethane, and 0.23 g of triethylamine (2.29 mmols) were dropwise added thereto while cooling the suspension with ice. Subsequently, a catalytic amount of 4-dimethylaminopyridine was added thereto. The reaction mixture was heated to room temperature, and stirred as such for 40 minutes. Then, 0.05 g of 6-chloronicotinoyl chloride (0.28 mol) were added thereto, and the mixture was stirred for 17 hours. 50 ml of dichloromethane and 50 ml of water were added to the reaction mixture. The dichloromethane layer was dried over anhydrous sodium sulfate, and the solvent was distilled off under reduced pressure. The resulting crystals were washed with hexane, and collected by filtration to obtain 0.62 g of the intended O-(6-chloronicotinoyl)-4-(α-chloroformaldoxime)-5-chloro-1-methyl-3-trifluoromethylpyrazole.

melting point: 116 to 117 °C

Example 7

Synthesis of Compound No. 3-8 of the Present Invention

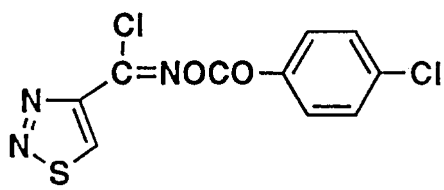

(1) Synthesis of 4-(α-chloroformaldoxime)-1,2,3-thiadiazole:

1.29 g of 1,2,3-thiadiazole-4-formaldoxime (10 mmols) was suspended in 50 ml of dichloromethane. While cooling the suspension with ice, 1.3 g of t-butyl hypochlorite (12 mmols) were dropwise added thereto. Then, the mixture was heated to room temperature, and stirred as such for 30 minutes. The solvent was distilled off under reduced pressure, and the thus-obtained crystals were washed with hexane to give 1.2 g of 4-(α-chloroformaldoxime)-1,2,3-thiadiazole as

brown crystals.
melting point: 132 to 133 °C

(2) Synthesis of Compound No. 3-8 of the Present Invention

0.5 g of 4-(α-chloroformaldoxime)-1,2,3-thiadiazole (3.06 mmols) and 0.59 g of 4-chlorobenzoyl chloride (3.36 mmols) were suspended in 40 ml of dichloromethane, and 0.37 g of triethylamine (3.67 mmols) were dropwise added thereto while stirring the suspension. Further, a catalytic amount of 4-dimethylaminopyridine was added thereto. The mixture was stirred as such for 4 hours. Subsequently, 100 ml of dichloromethane and 60 ml of water were added to the reaction mixture. The dichloromethane layer was dried overnight over anhydrous sodium sulfate and anhydrous magnesium sulfate, and was filtered under reduced pressure using Celite to remove insoluble matters. Further, the solvent was distilled off under reduced pressure. The thus-obtained crystals were washed with a mixed solvent of isopropyl ether and methanol (5 : 1), and were collected by filtration to obtain 0.4 g of the intended O-(4-chlorobenzoyl)-4-(α-chloroformaldoxime)-1,2,3-thiadiazole as colorless crystals
melting point: 173 to 174 °C

Example 8

Synthesis of Compound No. 1-59 of the Present Invention

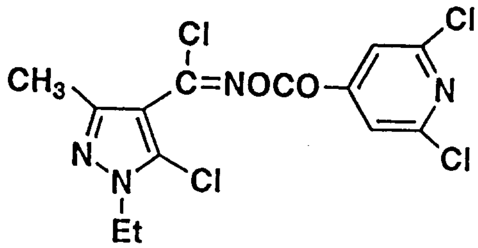

(1) Synthesis of 5-chloro-4-(α-chloroformaldoxime)-1-ethyl-3-methylpyrazole:

4.4 g of 5-chloro-1-ethyl-3-methylpyrazole-4-formaldoxime (25.5 mmols) was suspended in 50 ml of dichloromethane, and 3.3 g of t-butyl hypochlorite (30.4 mmols) were dropwise added thereto while cooling the suspension with ice. The mixture was stirred as such for 1 hour. The solvent was distilled off under reduced pressure, and the thus-obtained crystals were washed with hexane to obtain 4.0 g of the intended 5-chloro-4-(α-chloroformaldoxime)-1-ethyl-3-methylpyrazole as colorless crystals.
melting point: 120 to 122 °C

(2) Synthesis of Compound No. 1-59 of the Present Invention

0.6 g of 5-chloro-4-(α-chloroformaldoxime)-1-ethyl-3-methylpyrazole (2.9 mmols) and 0.67 g of 2,6-dichloropyridine-4-carbonyl chloride (3.18 mmols) were dissolved in 30 ml of dichloromethane, and 0.35 g of triethylamine (3.47 mmols) were dropwise added thereto while cooling the solution with ice. The reaction mixture was heated to room temperature, and stirred as such for 30 minutes.

30 ml of dichloromethane and 50 ml of water were added to the reaction mixture. The dichloromethane layer was dried over anhydrous sodium sulfate, and the solvent was distilled off under reduced pressure. The thus-obtained crystals were washed with hexane, and collected by filtration to obtain 0.7 g of the intended O-(2,6-dichloropyridine-4-carbonyl)-5-chloro-4-(α-chloroformaldoxime)-1-ethyl-3-methylpyrazole.
melting point: 123 to 124 °C

The compounds produced according to these methods are shown in Tables 4 to 6.

Table 4

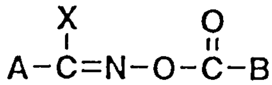

| No. | A | X | B | melting point ( ℃ ) |
|---|---|---|---|---|
| 1-1 | A 1 | C 1 | B 1 | 143.0-144.0 |
| 1-2 | A 1 | C 1 | B 2 | 134.0-136.0 |
| 1-3 | A 1 | C 1 | B 3 | 153.0-154.0 |
| 1-4 | A 2 | C 1 | B 1 | 120.0-122.0 |
| 1-5 | A 2 | C 1 | B 2 | 121.0-123.0 |
| 1-6 | A 2 | C 1 | B 3 | 172.0-174.0 |
| 1-7 | A 3 | C 1 | B 1 | 130.0-131.0 |
| 1-8 | A 3 | C 1 | B 2 | 159.0-161.0 |
| 1-9 | A 3 | C 1 | B 3 | 165.0-168.0 |
| 1-10 | A 1 | C 1 | B 4 | 132.0-134.0 |
| 1-11 | A 1 | C 1 | B 1 5 | 182.0-183.0 |
| 1-12 | A 4 | C 1 | B 1 | 117.0-118.0 |
| 1-13 | A 4 | C 1 | B 2 | 129.0-130.0 |
| 1-14 | A 4 | C 1 | B 3 | 144.0-145.0 |
| 1-15 | A 9 | C 1 | B 1 | 111.0-112.0 |
| 1-16 | A 9 | C 1 | B 2 | 162.0-163.0 |
| 1-17 | A 9 | C 1 | B 3 | 161.0-162.0 |

Table 4 (continued)

| No. | A | X | B | melting point ( ℃ ) |
|---|---|---|---|---|
| 1-18 | A 9 | C 1 | B 1 0 | 123.0-126.0 |
| 1-19 | A 9 | C 1 | B 1 3 | 136.0-137.0 |
| 1-20 | A 1 0 | C 1 | B 1 | 121.0-122.0 |
| 1-21 | A 1 0 | C 1 | B 2 | 150.0-151.0 |
| 1-22 | A 1 0 | C 1 | B 1 0 | 142.0-143.0 |
| 1-23 | A 1 1 | C 1 | B 1 | 96.0- 98.0 |
| 1-24 | A 1 1 | C 1 | B 2 | 121.0-124.0 |
| 1-25 | A 1 1 | C 1 | B 1 0 | 116.0-117.0 |
| 1-26 | A 1 2 | C 1 | B 1 | 154.0-155.0 |
| 1-27 | A 1 2 | C 1 | B 2 | 146.0-148.0 |
| 1-28 | A 1 2 | C 1 | B 1 0 | 184.0-187.0 |
| 1-29 | A 1 7 | C 1 | B 1 0 | 209.0-210.0 |
| 1-30 | A 1 8 | C 1 | B 1 0 | 125.0-128.0 |
| 1-31 | A 5 | C 1 | B 1 | 153.0-154.0 |
| 1-32 | A 5 | C 1 | B 2 | 156.0-157.0 |
| 1-33 | A 5 | C 1 | B 3 | 212.0-213.0 |
| 1-34 | A 9 | C 1 | B 7 | 82.0-83.0 |
| 1-35 | A 9 | C 1 | B 8 | 119.0-120.0 |
| 1-36 | A 9 | C 1 | B 9 | 138.0-139.0 |
| 1-37 | A 9 | C 1 | B 1 4 | 154.0-156.0 |
| 1-38 | A 9 | C 1 | B 1 5 | 166.0-167.0 |
| 1-39 | A 9 | C 1 | B 1 9 | 129.0-130.0 |
| 1-40 | A 9 | C 1 | B 2 0 | 147.0-148.0 |

Table 4 (continued)

| No. | A | X | B | melting point ( ℃ ) |
|---|---|---|---|---|
| 1-41 | A 9 | C 1 | B 2 1 | 116.0-118.0 |
| 1-42 | A 9 | C 1 | B 2 2 | 186.0-188.0 |
| 1-43 | A 9 | $SCH_3$ | B 1 0 | 113.0-114.0 |
| 1-44 | A 1 1 | C 1 | B 1 3 | 94.0-97.0 |
| 1-45 | A 1 1 | C 1 | B 1 4 | 137.0-138.0 |
| 1-46 | A 1 5 | C 1 | B 1 | 199.0-201.0 |
| 1-47 | A 1 5 | C 1 | B 2 | 153.0-155.0 |
| 1-48 | A 1 5 | C 1 | B 1 0 | 135.0-137.0 |
| 1-49 | A 1 7 | C 1 | B 1 | 134.0-135.0 |
| 1-50 | A 1 7 | C 1 | B 2 | 135.0-136.0 |
| 1-51 | A 1 8 | C 1 | B 1 | 118.0-120.0 |
| 1-52 | A 1 9 | C 1 | B 1 | 114.0-116.0 |
| 1-53 | A 1 9 | C 1 | B 1 0 | 93.0-96.0 |
| 1-54 | A 1 9 | C 1 | B 1 3 | 87.0-90.0 |
| 1-55 | A 2 0 | C 1 | B 1 0 | 102.0-103.0 |
| 1-56 | A 2 0 | C 1 | B 1 3 | 97.0-98.0 |
| 1-57 | A 2 1 | C 1 | B 1 | 97.0-99.0 |
| 1-58 | A 2 1 | C 1 | B 1 3 | 93.0-95.0 |
| 1-59 | A 2 1 | C 1 | B 1 4 | 123.0-124.0 |
| 1-60 | A 2 1 | C 1 | B 2 1 | 79.0-80.0 |
| 1-61 | A 2 1 | C 1 | B 2 2 | 128.0-130.0 |
| 1-62 | A 2 4 | C 1 | B 1 0 | 121.0-122.0 |

Table 4 (continued)

| No. | A | X | B | melting point ( ℃ ) |
|---|---|---|---|---|
| 1-63 | A 2 4 | C 1 | B 1 3 | 134.0-135.0 |
| 1-64 | A 2 4 | C 1 | B 1 4 | 122.0-125.0 |
| 1-65 | A 1 | C 1 | B 2 5 | 144.0-146.0 |
| 1-66 | A 9 | C 1 | B 1 7 | 146.5-148.0 |
| 1-67 | A 9 | C 1 | B 2 5 | 158.0-159.0 |
| 1-68 | A 9 | C 1 | B 3 1 | 120.0-121.0 |
| 1-69 | A 9 | C 1 | B 3 4 | 124.0-125.0 |
| 1-70 | A 9 | C 1 | B 3 5 | 139.0-140.0 |
| 1-71 | A 9 | C 1 | B 3 7 | 130.0-131.0 |
| 1-72 | A 9 | C 1 | B 3 8 | 130.0-131.0 |
| 1-73 | A 9 | C 1 | B 3 9 | 114.0-115.0 |
| 1-74 | A 9 | C 1 | B 4 4 | 138.0 139.0 |
| 1-75 | A 1 0 | C 1 | B 1 3 | 131.0-132.0 |
| 1-76 | A 2 1 | C 1 | B 1 7 | 93.0- 95.0 |
| 1-77 | A 2 1 | C 1 | B 2 5 | 98.0- 99.0 |
| 1-78 | A 2 1 | C 1 | B 3 1 | 69.0- 71.0 |
| 1-79 | A 2 1 | C 1 | B 4 1 | 74.0- 76.0 |
| 1-80 | A 2 1 | C 1 | B 4 2 | 103.0-105.0 |
| 1-81 | A 2 1 | C 1 | B 4 4 | 87.0- 89.0 |
| 1-82 | A 2 2 | C 1 | B 1 | 80.0- 81.0 |
| 1-83 | A 2 2 | C 1 | B 1 0 | 81.0- 83.0 |
| 1-84 | A 2 2 | C 1 | B 1 3 | 82.0- 84.0 |
| 1-85 | A 2 2 | C 1 | B 1 4 | 139.0-140.0 |

Table 4 (continued)

| No. | A | X | B | melting point ( ℃ ) |
|---|---|---|---|---|
| 1-86 | A 2 2 | C 1 | B 1 7 | 81.0- 83.0 |
| 1-87 | A 2 2 | C 1 | B 2 5 | 144.0-146.0 |
| 1-88 | A 2 3 | C 1 | B 1 3 | 110.0-113.0 |
| 1-89 | A 2 3 | C 1 | B 1 4 | 75.0- 78.0 |
| 1-90 | A 2 5 | C 1 | B 1 | 86.0- 87.0 |
| 1-91 | A 2 5 | C 1 | B 1 0 | 112.5-114.0 |
| 1-92 | A 2 5 | C 1 | B 1 3 | 134.0-135.0 |
| 1-93 | A 2 5 | C 1 | B 1 4 | 166.0-167.0 |
| 1-94 | A 2 6 | C 1 | B 1 | oil |
| 1-95 | A 2 6 | C 1 | B 1 0 | 90.0- 93.0 |
| 1-96 | A 2 6 | C 1 | B 1 3 | 109.0-110.0 |
| 1-97 | A 2 6 | C 1 | B 1 4 | 114.0-117.0 |
| 1-98 | A 2 8 | C 1 | B 1 4 | 178.0-190.0 |
| 1-99 | A 2 9 | C 1 | B 1 3 | 49.0- 52.0 |
| 1-100 | A 3 3 | C 1 | B 1 0 | 194.0-195.0 |
| 1-101 | A 3 3 | C 1 | B 1 3 | 160.0-161.0 |
| 1-102 | A 3 7 | C 1 | B 1 | 118.0-119.0 |
| 1-103 | A 3 7 | C 1 | B 1 0 | 143.5-145.0 |
| 1-104 | A 3 7 | C 1 | B 1 3 | 137.5-138.0 |
| 1-105 | A 3 7 | C 1 | B 1 4 | 145.0-146.0 |
| 1-106 | A 3 7 | C 1 | B 2 5 | 140.0-141.0 |
| 1-107 | A 3 8 | C 1 | B 1 | 157.5-158.5 |
| 1-108 | A 3 8 | C 1 | B 1 3 | 147.0-148.0 |

Table 4 (continued)

| No. | A | X | B | melting point ( ℃ ) |
|---|---|---|---|---|
| 1-109 | A 3 8 | C 1 | B 1 4 | 99.0-106.0 |
| 1-110 | A 9 | C 1 | B 4 0 | 128.0-129.0 |
| 1-111 | A 9 | C 1 | B 4 3 | 154.0-157.0 |
| 1-112 | A 9 | C 1 | B 2 6 | 153.0-154.5 |
| 1-113 | A 9 | C 1 | B 3 2 | 73.0- 78.0 |
| 1-114 | A 9 | C 1 | B 3 5 | 144.0-145.0 |
| 1-115 | A 9 | C 1 | B 4 6 | 143.0-148.0 |
| 1-116 | A 2 1 | C 1 | B 3 3 | 84.0- 89.0 |
| 1-117 | A 2 1 | C 1 | B 3 5 | 94.5- 95.5 |
| 1-118 | A 2 3 | C 1 | B 2 2 | 77.0- 80.0 |
| 1-119 | A 2 4 | C 1 | B 2 2 | 143.0-144.0 |
| 1-120 | A 2 4 | C 1 | B 3 5 | 115.0-116.0 |
| 1-121 | A 2 6 | C 1 | B 2 2 | 147.0-151.0 |
| 1-122 | A 2 8 | C 1 | B 2 5 | 160.0-163.0 |
| 1-123 | A 3 7 | C 1 | B 3 5 | 151.0-155.0 |
| 1-124 | A 9 | C 1 | B 1 1 | 69.0- 70.0 |
| 1-125 | A 9 | C 1 | B 1 2 | 109.0-110.0 |
| 1-126 | A 9 | C 1 | B 2 3 | 146.0-147.0 |
| 1-127 | A 9 | C 1 | B 4 7 | 100.0-101.5 |
| 1-128 | A 8 | C 1 | B 1 4 | 105.5-106.0 |
| 1-129 | A 8 | C 1 | B 2 5 | 83.5- 85.0 |

Table 5

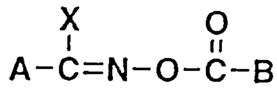

| No. | A | X | B | melting point ( ℃ ) |
|---|---|---|---|---|
| 2-1 | A 9 | C 1 | $CH_3$ | 48.0-49.0 |
| 2-2 | A 9 | C 1 | OEt | oil |
| 2-3 | A 1 2 | C 1 | $CH_3$ | 64.0-66.0 |
| 2-4 | A 9 | C 1 | $CH=CHCH_3$ (*) | semi-crystal |
| 2-5 | A 9 | C 1 | $CHBrC(CH_3)_3$ | oil |
| 2-6 | A 1 5 | C 1 | $CHBrC(CH_3)_3$ | oil |

* E-form

Table 6

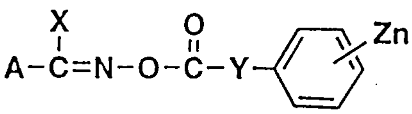

| No. | A | X | Y | Zn | melting point ( ℃ ) |
|---|---|---|---|---|---|
| 3-1 | A 9 | C 1 | – | 4-Cl | 104.0-105.0 |
| 3-2 | A 9 | C 1 | – | 4-$CF_3$ | 76.0-77.0 |
| 3-3 | A 9 | C 1 | – | 4-$NO_2$ | 152.0-153.0 |
| 3-4 | A 9 | C 1 | – | 3,5-$Cl_2$ | 160.0-161.0 |
| 3-5 | A 1 0 | C 1 | – | 4-$CF_3$ | 76.0-77.0 |
| 3-6 | A 1 1 | C 1 | – | 4-$CF_3$ | 75.0-77.0 |
| 3-7 | A 1 2 | C 1 | – | 4-$CF_3$ | 134.0-137.0 |
| 3-8 | A 1 7 | C 1 | – | 4-Cl | 173.0-174.0 |
| 3-9 | A 9 | C 1 | – | 4-F | 111.0-113.0 |
| 3-10 | A 9 | C 1 | – | 4-$OCH_3$ | 98.0-100.0 |
| 3-11 | A 9 | C 1 | – | 4-CN | 151.0-154.0 |
| 3-12 | A 9 | C 1 | – | 4-$CH_3$ | 87.0-88.0 |
| 3-13 | A 9 | C 1 | – | 3,4-$Cl_2$ | 106.0-109.0 |
| 3-14 | A 9 | C 1 | – | 3-$NO_2$-4-Cl | 141.0-143.0 |
| 3-15 | A 1 5 | C 1 | – | 4-Cl | 135.0-137.0 |
| 3-16 | A 1 7 | C 1 | – | 2,4-$Cl_2$ | 145.0-146.0 |
| 3-17 | A 1 8 | C 1 | – | 4-Cl | 140.0-145.0 |
| 3-18 | A 1 9 | C 1 | – | 4-Cl | 94.0-96.0 |

Table 6 (continued)

| No. | A | X | Y | Zn | melting point ( ℃ ) |
|---|---|---|---|---|---|
| 3-19 | A19 | Cl | – | 4-$NO_2$ | 122.0-125.0 |
| 3-20 | A21 | Cl | – | 4-CN | 122.0-124.0 |
| 3-21 | A24 | Cl | – | 4-Cl | 116.0-117.0 |
| 3-22 | A24 | Cl | – | 4-$NO_2$ | 151.0-152.0 |
| 3-23 | A9 | Cl | – | 2,6-$F_2$ | 103.5-104.5 |
| 3-24 | A25 | Cl | – | 4-$NO_2$ | 130.0-134.0 |
| 3-25 | A32 | Cl | – | 4-F | oil |

In the Tables 4 to 6, A1 to A41 and B1 to B47 have the same meanings as defined above.

In Table 7, main intermediates for synthesizing these compounds are shown.

Table 7

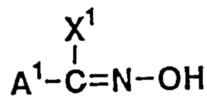

| No. | $A^1$ | $X^1$ | melting point (℃) |
|---|---|---|---|
| 4-1 | A 9 | C l | 140.0-143.0 (hydrochloride) |
| 4-2 | A 1 0 | C l | 134.0-135.0 |
| 4-3 | A 1 1 | C l | 99.0-101.0 |
| 4-4 | A 1 2 | C l | 124.0-126.0 |

Table 7 (continued)

| No. | $A^1$ | $X^1$ | melting point ( ℃ ) |
|---|---|---|---|
| 4-5 | A17 | Cl | 132.0-133.0 |
| 4-6 | A18 | Cl | 230.0-232.0 (hydrochloride) |
| 4-7 | A15 | Cl | 147.0-150.0 |
| 4-8 | A19 | Cl | 132.0-135.0 |
| 4-9 | A20 | Cl | 107.0-108.0 |
| 4-10 | A21 | Cl | 120.0-122.0 |
| 4-11 | A24 | Cl | 145.0-146.0 |
| 4-12 | A22 | Cl | 128.0-131.0 |
| 4-13 | A23 | Cl | 48.0- 50.0 |
| 4-14 | A25 | Cl | 134.0-135.0 |
| 4-15 | A26 | Cl | oil |
| 4-16 | A28 | Cl | 148.0-150.0 |
| 4-17 | A29 | Cl | 104.5-105.5 |
| 4-18 | A33 | Cl | 147.0-148.0 |
| 4-19 | A37 | Cl | 154.0-156.0 |
| 4-20 | A38 | Cl | 131.0-133.0 |
| 4-21 | A8 | Cl | 148.0-149.0 |

In the above-mentioned table, A8 to A38 are as defined above.

The usefulness of the compounds of the present invention will be described specifically by referring to the following Test Examples. However, it is not limited thereto.

Test Example 1

Test for effect of controlling Downy mildew (Pseudoperonospora cubensis):

A chemical solution prepared by diluting an emulsifiable concentrate of the compound of the present invention to 500 ppm with water was applied to a cucumber (type: Sagamihanpaku) at 1.5-leaf stage which was grown in a pot having 7 cm in diameter, in an amount of 20 ml per pot using a spray gun.

The following day, a suspension of spores of downy mildew (Pseudoperonospora cubensis) ($2 \times 10^5$ spores/ml) was sprayed onto the cucumber, and the cucumber was placed in an inoculation box kept at a temperature of 25 °C and a humidity of 95% or more for 24 hours. Then, the thus-treated cucumber was put in a greenhouse. After 7 days of inoculation, a ratio of an area of an infection formed to the inoculated leaf was measured, and a protective value was calculated according to the following formula.

$$\text{Protective value} = [1 - (A/B)] \times 100$$

(A: area ratio of infected leaves in treated group,

B: area ratio of infected leaves in control group)

Consequently, the following compounds showed the protective value of 100.

Present Compounds No. 1-1, No. 1-2, No. 1-3, No. 1-4, No. 1-5, No. 1-6, No. 1-7, No. 1-8, No. 1-9, No. 1-10, No. 1-11, No. 1-12, No. 1-13, No. 1-14, No. 1-18, No. 1-19, No. 1-20, No. 1-21, No. 1-22, No. 1-23, No. 1-24, No. 1-25, No. 1-29, No. 1-31, No. 1-32, No. 1-36, No. 1-37, No. 1-39, No. 1-40, No. 1-41, No. 1-42, No. 1-44, No. 1-45, No. 1-48, No. 1-49, No. 1-50, No. 1-52, No. 1-53, No. 1-54, No. 1-55, No. 1-56, No. 1-57, No. 1-58, No. 1-59, No. 1-60, No. 1-61, No. 1-63, No. 1-64, No. 1-65, No. 1,66, No. 1-67, No. 1-68, No. 1-70, No. 1-71, No. 1-74, No. 1-75, No. 1-76, No. 1-77, No. 1-79, No. 1-80, No. 1-81, No. 1-82, No. 1-83, No. 1-84, No. 1-85, No. 1-86, No. 1-87, No. 1-88, No. 1-89, No. 1-93, No. 1-94, No. 1-95, No. 1-96, No. 1-97, No. 1-98, No. 1-101, No. 1-102, No. 1-103, No. 1-104, No. 1-105, No. 1-106, No. 1-107, No. 1-109, No. 1-110, No. 1-111, No. 3-3, No. 3-4, No. 3-5 and No. 3-8.

Test Example 2

Test for effect of controlling Powdery mildew (Sphaerotheca fuliginea):

A chemical solution prepared by diluting an emulsifiable concentrate of the compound of the present invention to 500 ppm with water was sprayed onto a cucumber (type: Sagamihanpaku) at 1.5-leaf stage which was grown in a pot having 7 cm in diameter, in an amount of 20 ml per pot using a spray gun.

The following day, a suspension of spores of powdery mildew (Sphaerotheca fuliginea) ($3 \times 10^5$ spores/ml) was sprayed onto the cucumber, and inoculated. The thus-treated cucumber was then put in a greenhouse. After 10 days of inoculation, a ratio of an area of an infection formed to the inoculated leaf was measured, and a control value was calculated according to the following formula.

$$\text{Protective value} = [1 - (A/B)] \times 100$$

(A: area ratio of infected leaves in treated group,

B: area ratio of infected leaves in control group)

Consequently, the following compounds showed the protective value of 100.

Present Compounds No. 1-7, No. 1-10 and No. 1-59.

Test Example 3

Test for controlling Blast (Pyricularia oryzae):

A chemical solution prepared by diluting an emulsifiable concentrate of the compound of the present invention to 500 ppm with water was sprayed onto a rice (type: Nihonbare) at 3-leaf stage which was grown in a pot having 7 cm in diameter, in an amount of 20 ml per pot using a spray gun.

The following day, a suspension of spores of blast (Pyricularia oryzae) ($3 \times 10^5$ spores/ml) was sprayed onto the rice, and inoculated. The rice was placed in an inoculation box kept at a temperature of 25 °C and a humidity of 95% or

more for 24 hours. Then, the thus-treated rice was put in a greenhouse. After 7 days of inoculation, a ratio of an area of an infection formed was measured, and a protective value was calculated according to the following formula.

Protective value = [1 - (A/B)] x 100

(A: area ratio of infected leaves in treated group,

B: area ratio of infected leaves in control group)

Consequently, the following compounds showed the protective value of 100.

Present Compounds No. 1-1, No. 1-2, No. 1-3, No. 1-4, No. 1-5, No. 1-6, No. 1-7, No. 1-12, No. 1-14, No. 1-15, No. 1-16, No. 1-17, No. 1-19, No. 1-24, No. 1-37, No. 1-41, No. 1-42, No. 1-44, No. 1-45, No. 1-47, No. 1-48, No. 1-51, No. 1-52, No. 1-55, No. 1-56, No. 1-57, No. 1-59, No. 1-60, No. 1-61, No. 1-62, No. 1-63, No. 1-64, No. 1-65, No. 1-66, No. 1-67, No. 1-68, No. 1-69, No. 1-70, No. 1-71, No. 1-72, No. 1-73, No. 1-74, No. 1-75, No. 1-76, No. 1-77, No. 1-78, No. 1-79, No. 1-80, No. 1-81, No. 1-82, No. 1-83, No. 1-84, No. 1-85, No. 1-86, No. 1-87, No. 1-88, No. 1-89, No. 1-90, No. 1-93, No. 1-100, No. 1-102, No. 1-103, No. 1-104, No. 1-105, No. 1-106, No. 1-107, No. 1-108, No. 1-109, No. 3-2, No. 3-3, No. 3-4, No. 3-6, No. 3-8, No. 3-24 and No. 3-25.

Test Example 4

Test for controlling Blast (Pyricularia oryzae) (drench treatment):

A chemical solution prepared by diluting an emulsifiable concentrate of the compound of the present invention to 500 ppm with water was drenched on a rice (type: Nihonbare) at 1.5-leaf stage which was grown in a pot having 7 cm in diameter, in an amount of 10 ml per pot.

After 7 days of drench, a suspension of spores of blast (Pyricularia oryzae) (2 x $10^5$ spores/ml) was sprayed onto the rice, and inoculated. The rice was placed in an inoculation box kept at a temperature of 25 °C and a humidity of 95% or more for 24 hours. Then, the rice was put in a greenhouse. After 7 days of inoculation, a ratio of an area of an infection formed was measured, and a protective value was calculated according to the following formula.

Protective value = [1 - (A/B)] x 100

(A: area ratio of infected leaves in treated group,

B: area ratio of infected leaves in control group)

Consequently, the following compounds showed the protective control value of from 70 to 100.

Present Compounds No. 1-66, No. 1-67, No. 1-70, No. 1-75, No. 1-76, No. 1-77, No. 1-79, No. 1-80, No. 1-82, No. 1-84, No. 1-86, No. 1-87, No. 1-88, No. 1-89, No. 1-90, No. 1-91, No. 1-92, No. 1-93, No. 1-99, No. 1-101, No. 1-102, No. 1-103, No. 1-104, No. 1-105, No. 1-106, No. 1-107, No. 1-108, No. 1-109 and No. 3-25.

Test Example 5

Test for controlling Leaf rust (Puccinia recondita):

A chemical solution prepared by diluting an emulsifiable concentrate of the compound of the present invention to 500 ppm with water was applied to a wheat (type: Nohrin No. 61) at 1.5 to 2.0-leaf stage which was grown in a pot having 5.5cm in diameter, in an amount of 20 ml per pot using a spray gun.

The following day, a suspension of spores of Puccinia recondita (2 x $10^5$ spores/ml) was sprayed onto the wheat, and inoculated. The thus-inoculated wheat was placed in an inoculation box kept at a temperature of 25 °C and a humidity of 95% or more for 24 hours. Then, the wheat was put in a greenhouse. After 7 days of inoculation, a ratio of an area of an infection formed was measured, and a protective value was calculated according to the following formula.

Protective value = [1 - (A/B)] x 100

(A: area ratio of infected leaves in treated group,

B: area ratio of infected leaves in control group)

Consequently, the following compounds showed the control value of from 70 to 100.
Invention Compounds No. 1-76, No. 1-86, No. 1-103, No. 1-104, No. 1-105 and No. 1-106.

Test Example 6

Test for effect of controlling Gray mold (Botrytis cinerea):

A chemical solution prepared by diluting an emulsifiable concentrate of the compound of the present invention to 500 ppm with water was applied to a cucumber (type: Sagamihanpaku) at 1- to 2-leaf stage which was grown in a pot 7 cm in diameter, in an amount of 20 ml per pot using a spray gun.

The following day, a leaf was cut from the thus-applied cucumber, and was placed in a vat on which a paper soaked with water was laid. A lawn disc (4 cm in diameter) of gray mold (Botrytis cinerea) which had been cultured in a PSA medium was inoculated therein. After the completion of the inoculation, the vat was covered with a vinyl polymer sheet, and put in a constant-temperature chamber kept at 18°C for 5 days. A diameter of an infection formed was measured, and a protective value was calculated according to the following formula.

$$\text{Protective value} = [1 - (A/B)] \times 100$$

(A: diameter of infected leaves in treated group (mm),

B: diameter of infected leaves in control group (mm))

Consequently, the following compounds showed the protective value of 100.
Present Compounds No. 1-1, No. 1-3, No. 1-7, No. 1-11 and No. 1-25.

Industrial Availability

The compounds of the present invention are novel compounds which exhibit excellent agricultural and horticultural bacterial activity and which are free from phytotoxicity to useful crops. Accordingly, the compounds of the present invention are useful as agricultural and horticultural bactericides or active ingredients thereof.

## Claims

1. Aldoxime derivatives of formula [1]:

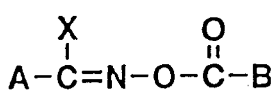

[1]

wherein X represents a halogen atom or $S(O)_nR^{24}$,
n is 0 or an integer of 1 to 2,
$R^{24}$ represents a $C_1$-$C_6$ alkyl group or an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),
A represents

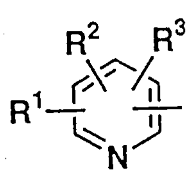 , 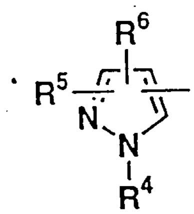 , 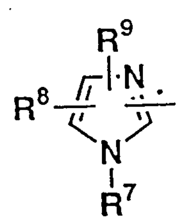 ,

A-1 A-2 A-3

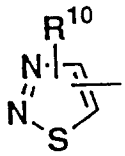

or

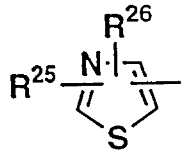

A-4

A-5

$R^4$ and $R^7$ independently represent a $C_1$-$C_6$ alkyl group, a $C_2$-$C_4$ alkenyl group, a $C_2$-$C_4$ alkynyl group, a $C_1$-$C_6$ haloalkyl group, an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), or an optionally substituted pyridyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 4, and the substituents may be the same or different),

$R^1$ to $R^3$, $R^5$, $R^6$, $R^8$ to $R^{1°}$, $R^{25}$ and $R^{26}$ independently represent a hydrogen atom, a halogen atom, a $C_1$-$C_6$ alkyl group, a $C_2$-$C_4$ alkenyl group, a $C_2$-$C_4$ alkynyl group, a $C_1$-$C_6$ haloalkyl group, a $C_1$-$C_6$ alkoxy group, a $C_1$-$C_6$ alkoxycarbonyl group, a cyano group, an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), or an optionally substituted phenoxy group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

B represents a $C_1$-$C_6$ alkyl group, a $C_2$-$C_6$ alkenyl group, a $C_2$-$C_6$ alkynyl group, a $C_1$-$C_6$ haloalkyl group, a $C_1$-$C_6$ alkoxy group, a $C_1$-$C_6$ alkylamino group, an optionally substituted $C_3$-$C_6$ cycloalkyl group (the substituent thereof is a halogen atom, a $C_1$-$C_6$ alkyl group, a $C_1$-$C_6$ haloalkyl group or a $C_1$-$C_6$ alkoxy group, the number of the substituents is between 1 and 3, and the substituents may be the same or different), an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), an optionally substituted phenoxy group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different), an optionally substituted phenylamino group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different

same or different),

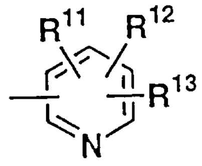 ,

B-1

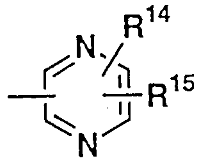 ,

B-2

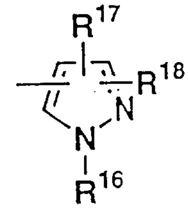 ,

B-3

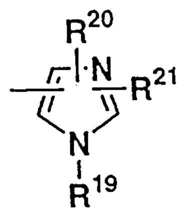 ,

B-4

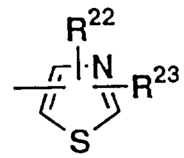 ,

B-5

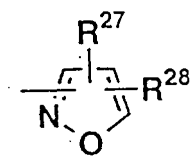 ,

B-6

or 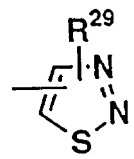 ,

B-7

$R^{16}$ and $R^{19}$ independently represent a $C_1$-$C_6$ alkyl group, a $C_2$-$C_4$ alkenyl group, a $C_2$-$C_4$ alkynyl group, a $C_1$-$C_6$ haloalkyl group or an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

$R^{11}$ to $R^{15}$, $R^{17}$, $R^{18}$, $R^{20}$ to $R^{23}$ and $R^{27}$ to $R^{29}$ independently represent a hydrogen atom, a halogen atom, a $C_1$-$C_6$ alkyl group, a $C_2$-$C_4$ alkenyl group, a $C_2$-$C_4$ alkynyl group, a $C_1$-$C_6$ haloalkyl group, a $C_1$-$C_6$ alkoxy group, an optionally substituted phenyl group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different) or an optionally substituted phenoxy group (the substituent thereof is a halogen atom, a $C_1$-$C_6$ alkyl group, a $C_1$-$C_6$ haloalkyl group, a $C_1$-$C_6$ alkoxy group, a cyano group or a nitro group, the number of the substituents is between 1 and 5, and the substituents may be the same or different),

provided that when the above-mentioned A is A-1, B is B-2, B-3, B-4, B-5, B-6 or B-7.

**2.** Aldoxime derivatives as claimed in Claim 1, wherein A represents A-2 or A-5.

**3.** Aldoxime derivatives as claimed in Claim 1, wherein A represents A-2 or A-5 and B represents B-1, B-3 or B-5.

**4.** Aldoxime derivatives of formula [1] wherein A is A-2, B is B-1, B-3 or B-5, $R^4$ and $R^{16}$ are each a $C_1$-$C_6$ alkyl group, $R^5$, $R^6$, $R^{11}$ to $R^{13}$, $R^{17}$, $R^{18}$, $R^{22}$, $R^{23}$, $R^{25}$ and $R^{26}$ are independently a hydrogen atom, a halogen atom, a $C_1$-$C_6$ alkyl group, a $C_1$-$C_6$ haloalkyl group or a $C_1$-$C_6$ alkoxy group.

**5.** Aldoxime derivatives as claimed in Claim 1, wherein A represents A-2 or A-5, B represents B-1, B-3 or B-5,
$R^4$ and $R^{16}$ represent each a $C_1$-$C_6$ alkyl group,
$R^5$, $R^6$, $R^{11}$ to $R^{13}$, $R^{17}$, $R^{18}$, $R^{22}$, $R^{23}$, $R^{25}$ and $R^{26}$ independently represent a hydrogen atom, a halogen atom, a

$C_1$-$C_6$ alkyl group, a $C_1$-$C_6$ haloalkyl group or a $C_1$-$C_6$ alkoxy group, and X represents a halogen atom.

**6.** Aldoxime derivatives as claimed in Claim 1, wherein A represents A-2, B represents B-1, $R^4$ represents a $C_1$-$C_6$ alkyl group, $R^5$, $R^6$ and $R^{11}$ to $R^{13}$ independently represent a hydrogen atom, a halogen atom or a $C_1$-$C_6$ alkyl group, and X represents a halogen atom.

**7.** Aldoxime derivatives as claimed in Claim 1, wherein A represents A-2, B represents B-3, $R^4$ and $R^{16}$ represent each a $C_1$-$C_6$ alkyl group, $R^5$, $R^6$, $R^{17}$ and $R^{18}$ independently represent a hydrogen atom, a halogen atom or a $C_1$-$C_6$ alkyl group, and X represents a halogen atom.

**8.** Aldoxime derivatives as claimed in Claim 1, wherein A represents A-2, B represents B-5, $R^4$ represents a $C_1$-$C_6$ alkyl group, $R^5$, $R^6$, $R^{22}$ and $R^{23}$ independently represent a hydrogen atom, a halogen atom or a $C_1$-$C_6$ alkyl group, and X represents a halogen atom.

**9.** Aldoxime derivatives as claimed in Claim 1, wherein A represents A-5, B represents B-1, $R^{11}$ to $R^{13}$, $R^{25}$ and $R^{26}$ independently represent a hydrogen atom, a halogen atom or a $C_1$-$C_6$ alkyl group, and X represents a halogen atom.

**10.** Aldoxime derivatives as claimed in Claim 1, wherein A represents A-5, B represents B-3, $R^{16}$ represents a $C_1$-$C_6$ alkyl group, $R^{17}$, $R^{18}$, $R^{25}$ and $R^{26}$ independently represent a hydrogen atom, a halogen atom or a $C_1$-$C_6$ alkyl group, and X represents a halogen atom.

**11.** Aldoxime derivatives as claimed in Claim 1, wherein A represents A-5, B represents B-5, $R^{22}$, $R^{23}$, $R^{25}$ and $R^{26}$ independently represent a hydrogen atom, a halogen atom or a $C_1$-$C_6$ alkyl group, and X represents a halogen atom.

**12.** Aldoxime derivatives comprising:

(1) O-(5,6-dichloronicotinoyl)-5-chloro-4-(α-chloroformaldoxime)-1,3-dimethylpyrazole,
(2) O-(2,6-dichloropyridine-4-carbonyl)-5-chloro-4-(α-chloroformaldoxime)-1-ethyl-3-methylpyrazole,
(3) O-(2-chloro-6-methylpyridine-4-carbonyl)-5-chloro-4-(α-chloroformaldoxime)-1-ethyl-3-methylpyrazole,
(4) O-(5,6-dichloronicotinoyl)-4-chloro-5-(α-chloroformaldoxime)-1,3-dimethylpyrazole,
(5) O-(5-chloronicotinoyl)-4-chloro-5-(α-chloroformaldoxime)-1,3-dimethylpyrazole,
(6) O-(1,3-dimethylpyrazole-5-carbonyl)-5-chloro-4-(α-chloroformaldoxime)-1,3-dimethylpyrazole,
(7) O-(3-chloro-1-methylpyrazole-5-carbonyl)-5-chloro-4-(α-chloroformaldoxime)-1-ethyl-3-methylpyrazole,
(8) O-(2-chloro-4-methylthiazole-5-carbonyl)-5-chloro-4-(α-chloroformaldoxime)-1,3-dimethylpyrazole, or
(9) O-(2,6-dichloropyridine-4-carbonyl)-5-(α-chloroformaldoxime)-2,4-dimethylthiazole.

**13.** Aldoxime derivatives of formula [2]:

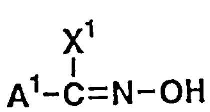

[2]

wherein $X^1$ represents a halogen atom, and $A^1$ represents A-2, A-3, A-4 or A-5 as defined in Claim 1.

**14.** Agricultural and horticultural bactericides containing one or more of aldoxime derivatives described in Claim 1.

## INTERNATIONAL SEARCH REPORT

International application No.
PCT/JP94/01041

**A. CLASSIFICATION OF SUBJECT MATTER**

Int. $Cl^5$ C07D213/81, 83, 233/90, 277/56, 417/12, 401/12, 403/12, 285/06, 231/16, A01N43/40, 50, 56, 60, 78, 80, 82

According to International Patent Classification (IPC) or to both national classification and IPC

**B. FIELDS SEARCHED**

Minimum documentation searched (classification system followed by classification symbols)

Int. $Cl^5$ C07D213/00-90, 231/00-56, 277/00-84, 285/00-38, 401/00-14, 403/00-14, 417/00-14, A01N43/00-92

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| Y | JP, A, 5-51363 (Nippon Bayer Agrochem K.K.), March 2, 1993 (02. 03. 93), Claim, (Family: none) | 1-14 |
| Y | JP, A, 5-51364 (Nippon Bayer Agrochem K.K.), March 2, 1993 (02. 03. 93), Claim, (Family: none) | 1-14 |
| Y | JP, A, 4-134071 (Nippon Bayer Agrochem K.K.), May 7, 1993 (07. 05. 93), Claim & EP, A, 477678 | 1-14 |
| Y | JP, A, 62-270561 (Bayer AG.), November 24, 1987 (24. 11. 87), Claim & EP, A, 236,897 & DE, A, 3,608.383 & US, A, 4742070 | 1-14 |
| Y | JP, A, 60-81104 (Nippon Tokushu Noyaku Seizo K.K.), May 9, 1985 (09. 05. 85), Claim, (Family: none) | 1-14 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| September 5, 1994 (05. 09. 94) | September 27, 1994 (27. 09. 94) |
| Name and mailing address of the ISA/ Japanese Patent Office | Authorized officer |
| Facsimile No. | Telephone No. |

Form PCT/ISA/210 (second sheet) (July 1992)